(19) Europäisches Patentamt
European Patent Office
Office européen des brevets

(11)  EP 2 581 846 A1

(12)  EUROPEAN PATENT APPLICATION

(43) Date of publication:
17.04.2013  Bulletin 2013/16

(51) Int Cl.:
*G06F 19/00* (2011.01)          *A61B 5/00* (2006.01)
*A61M 1/36* (2006.01)

(21) Application number: 11184981.6

(22) Date of filing: 13.10.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Heartware BVBA
3140 Keerbergen (BE)

(72) Inventor: Janssenswillen, Eddy
3140 KEERBERGEN (BE)

(74) Representative: Plas, Axel Ivo Michel
IP HILLS NV
Hubert Frère-Orbanlaan 329
9000 Gent (BE)

(54)  **A cardiopulmonary bypass or cpb monitoring tool**

(57)  A cardiopulmonary bypass or CPB monitoring tool (100) comprising:
- a preoperative information module (101);
- a preoperative calculation module (102) able to estimate a body surface area, blood volume, and theoretical weight;
- a priming module (103) able to determine priming constitution, volume and flow to achieve a hemodilution target;
- an operation risk module (105) for calculating operation risk;
- a drug calculation module (104) able to determine medication doses;
- a timer module (106) with timers that can be activated during operation;
- a data collection module (107) comprising an interface and drivers enabling data collection from a wide variety of extracorporeal pumps and oxygenators during operation;
- an events module (108) with retroactive manipulation of the time of an event;
- a printing report generation module (109);
- a graphic user interface (113); and
- a configuration module (114).

Fig. 1

EP 2 581 846 A1

**Description**

**Field of the Invention**

The present invention generally relates to a tool for monitoring a cardiopulmonary bypass or CPB, i.e. technology that temporarily takes over the function of heart and lungs during surgery through extracorporeal blood circulation and oxygenation. A simplified form of CPB taking over the function of heart and/or lungs during a longer period as life-support for newborns and adults, is also known as ExtraCorporeal Membrane Oxygenation or ECMO. The invention in particular concerns a tool for CPB monitoring that is universally compatible with numerous CPB pumps and other devices, that is intuitive and user-friendly to the perfusionists that use it, and that supports customized graphic representation of parameters, curves and printing reports in order to reduce the overall input effort required from busy perfusionists.

**Background of the Invention**

Cardiopulmonary bypass or CPB is used during heart surgery because of the difficulty of operating on a beating heart. Extracorporeal membrane oxygenation or ECMO, a simplified form of CPB, is used as a long term life-support technology. CPB mechanically circulates and oxygenates blood while bypassing the heart and lungs, thereby maintaining perfusion of other body organs and tissues. The surgeon typically places a cannula in the right atrium, vena cava or femoral vein to extract blood from the patients body. Venous blood extracted from the body via the cannula is filtered, cooled or warmed, oxygenated and returned to the patients body. This is done by a so called heart-lung machine, typically featuring two functional units: a pump and an extracorporeal oxygenator that remove oxygen-deprived blood from the patients body and replace it with oxygen-rich blood. The cannula used to return the blood is inserted in the ascending aorta, or femoral artery. The blood is administered heparin to prevent clotting. The components of the CPB are interconnected by tubes, typically made of silicone rubber or PVC.

Different tools exist for monitoring a CPB, i.e. hardware and/or software tools that operate as a data management system, collecting data from the CPB components, and interacting with the perfusionist operating the CPB equipment. These tools are usually proprietary tools, i.e. hardware specific tools that interface only with CPB components from one particular supplier, and these tools require significant input effort from the perfusionist because they are not or poorly customizable and non-intuitive to the perfusionist.

One example of an existing CPB monitoring tool is the Data Management System from Sorin whose datasheet can be retrieved from the Internet via the following URL:

http://www.sorin.com/sites/default/files/roles/5/files/Sorin_DMS Slick.pdf

As is mentioned in the datasheet of Sorin's DMS system, this tool is adapted to be mounted on and to interface with Sorin's perfusion system. In other words, like most CPB monitoring tools, Sorin's DMS is a proprietary tool that connects an interfaces only with Sorin's SIII, S5 and C5 heart-lung machines, i.e. pumps and oxygenators from a single manufacturer, requiring the medical team or hospital to use only heart-lung machines from a single manufacturer, or to use plural CPB monitoring tools that each generate different visuals, data entry screens, graphs, reports, etc. This heterogeneity is not desirable.

Spectrum Medical's VIPER product described by author A. Hart in the "VIPER INDEPENDENT DATA MANAGEMENT USER MANUAL" is a tool for CPB monitoring that is built on an internal data base receiving data from the connected CPB devices and input data from the perfusionist using VIPER's graphical user interface. VIPER contains appropriate data entry interfaces for preoperative information such as patient data including pathology and medication information (paragraph 2.0 in the manual), personnel data for the medical team (paragraph 2.2 in the manual), and disposables and equipment data eventually selected from a pre-set (paragraphs 2.5 and 2.6 in the manual). VIPER further contains a priming module enabling to define and select pre-set priming fluid constitution (paragraph 2.4 in the manual). This module predicts the hemodilution in function of the selected priming fluid constitution and volume entered by the perfusionist. A prime optimization function enables to determine the priming fluid necessary to achieve a hemodilution target, assuming the perfusionist has entered a priming constitution (e.g. a pre-set) and target HCT value. The priming fluid predictions are calculated from preoperation statistics, i.e. lab values received before the operation, and the patient data. VIPER further contains a live data collection module collecting data during operation such as information on events during operation entered by the perfusionist (paragraph 3.1 in the manual) and data collected from the connected CPB equipment through an RS232 or Ethernet interface (paragraph 5.2 in the manual) and collected either automatically or manually from various sensors (paragraphs 3.4 and 3.5 in the manual). In administration or configuration mode, the user of VIPER at last is enabled to configure which parameters are to be collected and recorded by VIPER during operation (paragraph 4.2 in the manual), to configure the frequency, resolution and page settings of printed charts (paragraph 4.4 in the manual).

Although VIPER is able to connect and interface with various heart-lung machines and consequently not tied to pumps or oxygenators from one particular manufacturer, it is still limitedly customizable as a result of which perfusionists cannot use it in an intuitive and user-friendly manner without assistance from IT personnel.

VIPER does not enable the perfusionist to select, label and position in GUI screens the fields for preoperative data collection, does not enable the perfusionist to configure screens that are displayed during operation, to configure printing reports (apart from some resolution and page settings) in such a manner that the hospital can maintain the reporting formats it was used to, and does not enable the perfusionist to retroactively configure or manipulate the timing of events in case these events took place or were reported at a point in time during operation where the perfusionist was too busy. VIPER further is disadvantageous in its accuracy and completeness, for instance not taking into account the patient's body surface area, age or gender in priming calculations, and not generating information indicative for the operation risk. It is not exploring derived calculated parameters in order to assist the perfusionist's decision-making during the procedure. The overall impression of VIPER for the perfusionist hence is a lack of flexibility and lack of dynamics in its user-configurability. In addition, VIPER fails to produce accurate and essential information to assist the perfusionist during the procedure, and is in fact just a data logger enabling to produce a report after treatment.

The above prior art solutions have additional drawbacks that are resolved by embodiments of the present invention, such as the inability of remotely monitoring of ECMO putting a burden on hospital personnel, the inability to determine parameters of importance in paediatric CPB, the inability to conveniently visualize the bypass prior operation and heparin dose response during operation, and the inability to generate statistics and use such statistics for instance for automated, evidence-based material selection.

It is an objective of the present invention to resolve the above listed drawbacks of existing prior art solutions. In particular, it is an objective of the present invention to present a CPB monitoring tool that is more intuitive and user-friendly to the perfusionist in terms of its configurability, dynamics in generating charts and printed reports, and graphical user interfacing. It is an additional objective to disclose such CPB monitoring tool with more reliable priming calculation, and further advanced features that render the same CPB monitoring tool also useful and convenient for ECMO and paediatric CPB. The objective of the present invention is to help and assist the perfusionist during the procedure instead of merely providing an automatic data gathering system to produce a database used after the procedure for report printing.

## Summary of the Invention

According to the present invention, the above identified objectives are realized by a cardiopulmonary bypass or CPB monitoring tool as defined by claim 1, the CPB monitoring tool comprising:

- a preoperative information module enabling entry and management of patient data, pathology data, medication data, operation team data, material data for use during operation;
- a preoperative calculation module able to estimate a body surface area or BSA, blood volume, and theoretical weight from the patient data;
- a priming module able to determine priming constitution, volume and flow to achieve a hemodilution target;
- an operation risk module for calculating operation risk according to Euroscore and/or Parsonnet formulae;
- a drug calculation module able to determine medication doses that must be administered during operation;
- a timer module comprising one or more timers that can be activated during operation;
- a data collection module comprising an interface and drivers enabling data collection from a wide variety of extracorporeal pumps and oxygenators during operation;
- an events module enabling entry and management of events during operation, the events module enabling retroactive manipulation of the time of an event;
- a printing report generation module with user-configurable parameter selection for at least one report;
- a graphic user interface; and
- a configuration module for the graphic user interface, the configuration module enabling selection of fields for entry of preoperative information, labelling of the fields selected and positioning of the fields selected in data entry screens used by the preoperative information module for entry of preoperative information, enabling configuration of standard priming constitutions, enabling initialisation of medical team members, enabling initialisation of materials, enabling configuration of interfaces to a wide variety of extracorporeal pumps, and enabling configuration of chart screens displayed during operation in the graphical user interface.

Thus, through a modular approach with a configuration module that enables the user/perfusionist to configure the data entry screens, chart screens and printing reports, the perfusionist can tune the CPB monitoring tool to request the preoperative data, display charts during operation and produce printed reports in a user-friendly, intuitive manner where the medical team in the hospital is used to and that is identical independent of the heart-lung machine hardware that is used. The CPB monitoring tool according to the invention further enables the perfusionist to adapt the timing of events, even after the operation, such that event logging becomes more accurate, even if the perfusionist is busy at the point in time where an event takes place. Further, the CPB monitoring tool according to the invention gains in accurateness for priming calculations because the software first estimates the body surface area, blood volume and theoretical weight of the patient from the preoperative patient data. Summarizing, the CPB monitoring tool according to the invention is generic in terms of its connectivity to a wide variety of heart-lung machinery from different vendors, and in addition provides an unmatched accurateness and configurability of GUI screens and reports to the perfusionist.

According to an optional aspect defined by claim 2, the priming module in the CPB monitoring tool according to the current invention may further be adapted to determine valve diameters and/or cannula sizes for paediatric CPB.

Thus, starting from the body surface area, the priming module determines the size of the valves for paediatric CPB. For children up to 14 years, the size of four valves is calculated as follows

$$y_{PV} = 4.9706 \ln(x) + 15.298$$

$$y_{MV} = 6.3372 \ln(x) + 20.188$$

$$y_{TV} = 8.2593 \ln(x) + 24.861$$

$$y_{AV} = 4.7349 \ln(x) + 13.905$$

Herein,
$y_{PV}$ represents the pulmonary valve diameter;
$y_{MV}$ represents the mitral valve diameter;
$y_{TV}$ represents the tricuspide valve diameter;
$y_{AV}$ represents the aortic valve diameter; and
x represents the body surface area or BSA expressed in $m^2$.

According to a further aspect of the CPB monitoring tool according to the present invention, defined by claim 3, the timer module may comprise:

- a first timer for registering bypass time;
- a second timer for registering aorta clamp time;
- a third timer for registering time lapsed since a last Anti Coagulation Time or ACT measurement; and
- a fourth timer for registering time lapsed since a last Cardioplegia or CPG dose.

Thus, the timing module may contain at least four chronometers for measuring the bypass time, the aorta clamp time, the ACT time and CPG time. When the aorta clamp timer is stopped and the bypass timer is not stopped, the recirculation time is seen. The ACT timer shows the time elapsed since the last ACT measurement and automatically restarts after entering a new ACT value. The CPG timer shows the time elapsed since the last CPG dose and automatically restarts after entereing a new CPG amount.

As is further specified by claim 4, the timer module may comprise one or more user-configurable timers.

Indeed, these user configurable timers may be labelled and used according to the perfusionist's preferences, creating another degree of flexibility.

Optionally, as is defined by claim 5, the events module in the CPB monitoring tool according to the present invention stores a list of standard events that take place before, during and after a PCB.

Indeed, a list of standard events in the CPB procedure may be preconfigured, such as for instance "Patient in the waiting room", "Induction anaesthesia", "Patient ready", etc.

Optionally, as defined by claim 6, the CPB monitoring tool according to the current invention may further comprise:

- a medication module adapted to log medication supplied during operation.

Indeed, the CPB monitoring tool shall log medication administered during operation. The medication is entered or selected from a list, and the dose administered can be entered by the medical team in various units.

According to another optional aspect defined by claim 7, the CPB monitoring tool according to the invention further comprises:

- a theoretical and measured haematocrit evolution graph generator enabling to monitor the evolution of the patients hemodilution throughout the procedure.

Graphs displaying the evolution of the in-line haematocrit, the evolution of the theoretically calculated haematocrit, and the haemoglobin values measured through gasometry give the perfusionist a better view on the evolution of the oxygen transport capacity of the circulating blood. These graphs consequently shall enable the perfusionist to take better founded decisions based on accurate up-to-date information.

According to yet another optional aspect defined by claim 8, the CPB monitoring tool according to the invention further comprises:

- a heparin dose response curve generator enabling to derive the patients response to a first heparin dose and to predict additional heparin doses in order to achieve a target ACT value and to predict at the end of an operation procedure how much heparin is leftover to be neutralized in order to restore normal coagulation.

This curve represents the patient's individual reaction to a specific amount of heparin administered and can be drawn when the ACT value before heparin supply, the first heparin dose, and the ACT value after supply of the first heparin dose are entered into the CPB monitoring tool. Knowledge of this individual reaction can then be used to determine the extra heparin that is needed to reach a target ACT value.

Further optionally, as defined by claim 9, the CPB monitoring tool according to the current invention may comprise:

- a draw module enabling drawing a coronary bypass and sequential anastomosis.

Indeed, advantageously the CPB monitoring tool contains a drawing program that enables to visualize e.g. up to six bypasses, and to indicate a sequential anastomosis. The drawing module may further assist in selecting and memorizing the materials used for the bypass.

As is further defined by claim 10, the material module in the CPB monitoring tool according to the current invention may be adapted for evidence based material selection.

Such evidence based material selection maps the patient to other patients memorized in a database, determines the deviation from these patients, and determines which materials are best used for the patient in function of materials that were used with the best matching patients in the database.

According to yet another option defined by claim 11, the CPB monitoring tool according to the current invention may comprise:

- a statistical module for statistic calculations on a population of patients.

The statistical module is a server application that enables to select a population of patients through exclusion/inclusion criteria, e.g. starting and ending dates, blood group(s), gender, etc. The statistical module further enables to select the parameters or data that will be exported. The exported data are then used to generate graphs visualizing all kinds of statistics for the selected population of patients enabling to assist researchers in developing taxonomies, to discover structures and associations in data.

An embodiment of the CPB monitoring tool according the current invention, defined by claim 12, further comprises:

- connectivity to an application enabling remote monitoring during extracorporeal membrane oxygenation or ECMO.

Thus, the CPB hardware may be used for extracorporeal membrane oxygenation or ECMO in combination with an embodiment of the CPB monitoring tool according to the present invention that supports remote take-over of the screen, e.g. on a smartphone, tablet PC or laptop. Thereto, the CPB monitoring tool contains software that establishes connectivity to a wide area wireless network, e.g. a 3G network, and the GUI screens generated by the CPB monitoring tool are made available via a wireless connection to a CPB monitoring application installed on the user's mobile device. This way, the perfusionist or other medical personnel need not be present during the 24 hour or 48 hour ECMO heart assist.

Further, as defined by claim 13, the CPB monitoring tool according to the invention may comprise:

- a module for alarm generation through e-mail or SMS.

Hence, on top of remote take-over of the screen, the perfusionist or medical personal may be informed regularly, e.g. every 3 hours, on the ECMO patient's status, and/or alarm generating SMS or e-mail messages may be sent when certain events take place.

As defined by claim 14, the CPB monitoring tool according to the current invention is further adapted to visualize during operation derived calculated parameters to assist a perfusionist.

Indeed, derived calculated parameters like oxygen consumption and systemic vascular resistance curves are continuously visualised in order to assist the perfusionist during the procedure. The absolute minimum blood flow needed to assure vital oxygen delivery is constantly calculated taking into account the temperature, hemodilution and morphology of the patient. The actual cardiac index (blood flow per $m^2$) is constantly visualised. The temperature difference between patient temperature and blood temperature is monitored and shown in order to alert the perfusionist when temperature gradients become too large. The in-line pressure differences measured before and after the membrane oxygenator are constantly shown to be evaluated by the perfusionist during the procedure and to alert him in case of overpressure. All this among other features makes the tool according to the present invention much more than just a data logger to produce a database, but a real monitor assisting the perfusionist during the procedure and enabling him to make better founded decisions throughout the whole procedure.

**Brief Description of the Drawings**

Fig. 1 is a functional block scheme of an embodiment of the CPB monitoring tool 100 according to the present invention;

Fig. 2 shows a GUI screen 200 for entry of preoperative data in the embodiment 100 of the CPB monitoring tool according to the present invention;

Fig. 3 shows a GUI screen 300 illustrating preoperative calculations in the embodiment 100 of the CPB monitoring tool according to the invention;

Fig. 4 shows a GUI screen 400 for entry of team data in the embodiment 100 of the CPB monitoring tool according to the present invention;

Fig. 5 shows a GUI screen 500 for entry of material data in the embodiment 100 of the CPB monitoring tool according to the present invention;

Fig. 6 shows a GUI screen 600 for entry of pathology data in the embodiment 100 of the CPB monitoring tool according to the present invention;

Fig. 7 shows a GUI screen 700 for entry of medication data in the embodiment 100 of the CPB monitoring tool according to the present invention;

Fig. 8 shows a GUI screen 800 illustrating operation risk calculation in the embodiment 100 of the CPB monitoring tool according to the present invention;

Fig. 9 shows a GUI screen 900 illustrating drug calculation in the embodiment 100 of the CPB monitoring tool according to the present invention;

Fig. 10 shows a GUI screen 1000 displaying timers in the embodiment 100 of the CPB monitoring tool according to the present invention;

Fig. 11 shows a GUI screen 1100 for entry of event data in the embodiment 100 of the CPB monitoring tool according to the present invention;

Fig. 12 shows a GUI screen 1200 for entry of data related to medication administered during CPB in the embodiment 100 of the CPB monitoring tool according to the present invention;

Fig. 13 shows a GUI screen 1300 illustrating Ht-Hb graph generation in the embodiment 100 of the CPB monitoring tool according to the present invention;

Fig. 14 shows a GUI screen 1400 illustrating heparin dose response curve generation in the embodiment 100 of the CPB monitoring tool according to the present invention;

Fig. 15 shows a GUI screen 1500 illustrating operation of a drawing module in the embodiment 100 of the CPB monitoring tool according to the present invention;

Fig. 16 shows a GUI screen 1600 illustrating configuration of the preoperative data screen 200 in the embodiment 100 of the CPB monitoring tool according to the present invention;

Fig. 17 shows a GUI screen 1700 illustrating configuration of standard priming constitution in the embodiment 100 of the CPB monitoring tool according to the present invention;

Fig. 18 shows a GUI screen 1800 illustrating configuration of the medical team data screen 400 in the embodiment 100 of the CPB monitoring tool according to the present invention;

Fig. 19 shows a GUI screen 1900 illustrating configuration of the material data screen 500 in the embodiment 100 of the CPB monitoring tool according to the present invention;

Fig. 20 shows a GUI screen 2000 illustrating configuration of the data collection interface in the embodiment 100 of the CPB monitoring tool according to the present invention;

Fig. 21 shows a GUI screen 2100 illustrating statistics generation in the embodiment 100 of the CPB monitoring tool according to the present invention;

Fig. 22 shows a GUI screen 2200 shown during operation by the embodiment of the CPB monitoring tool 100 according to the present invention;

Fig. 23 illustrates in more detail the visualization of derived parameters in GUI screen 2200; and

Fig. 24 illustrates in more detail visualization of the minimum blood flow needed to transport oxygen to the whole body in GUI screen 2200.

## Detailed Description of Embodiment(s)

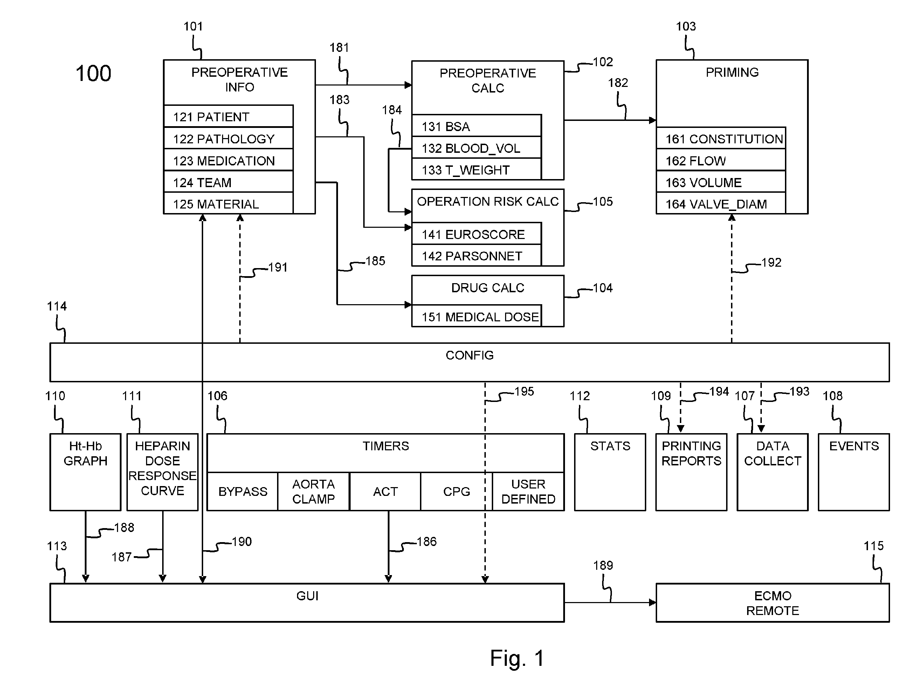

Fig. 1 shows the functional blocks of an embodiment 100 of the CPB monitoring tool according to the present invention. The preoperative information block 101 collects preoperative information, including patient data 121, pathology data 122, medication data 123, medical team data 124 and information on the materials used during operation 125. The preoperative information is collected from the user through interaction via various GUI screens that can be preconfigured by the user / perfusionist through the configuration module 114, as is indicated by arrow 191. The preoperative information, or portions thereof are communicated to the preoperative calculation block 102, as is indicated by arrow 181. This preoperative calculation method 102 calculates a number of parameters including the patient's body surface area or BSA 131, the patient's blood volume or BLOOD_VOL 132, and the patient's theoretical weight or T_WEIGHT 133. A few formula's that can be used to calculate these parameters from for instance the patient data 121 will be listed further below. The preoperative information and certain parameters calculated by the preoperative calculation block 102 are further communicated to the operation risk calculation block 105, as is indicated by arrows 183 and 184 in Fig. 1. The operation risk calculator 105 calculates the operation risk for the patient according to Euroscore formulae 141 and/or Parsonnet formulae 142. The preoperative information or portions thereof are further communicated to the drug calculation block 104, as is indicated by arrow 185, in order to enable the drug calculator 104 to determine the medical doses 151 to be administered. Still prior to operation, priming module 103 determines the constitution 161, flow 162 and volume 163 of the priming fluid that will be used during operation. The priming module 103 thereto uses the parameter values determined by the preoperative calculation block 102, as is indicated by arrow 182. The priming module 103 may use preconfigured priming sets, as is indicated by arrow 192. This will be explained in more detail further below. The priming module 103 or the preoperative calculation block 102 may further determine the valve diameters 164. This is of particular importance in case of paediatric CPB. The embodiment 100 illustrated by Fig. 1 further contains a timers module 106 at least comprising a chronometer for the bypass time, a chronometer for the aorta clamp time, a chronometer measuring the time since the last ACT, a chronometer measuring the time since the last CPG dose, and one or more user configurable timers. The embodiment 100 further also contains a data collection module 107 that interfaces with the heart-lung machine and eventual other devices to collect various sensed parameters such as temperatures, pressures, etc. The interfacing and the parameter values that are collected are configured by the user through the configuration module 114 as is indicated by arrow 193. Similarly, the user can configure the contents, structure and layout of printing reports that are produced by printing reports module 109. This is indicated by arrow 194 in Fig. 1. Events module 108 enables the user to enter event information prior to and during operation. The timing of these events can be adjusted retroactively which is advantageous in case the perfusionist is busy for instance at the point in time an event takes place. The Ht-Hb graph generator 110 enables the perfusionist to monitor the evolution of the hemodilution. The heparin dose response curve generator 111 visualizes the patient's individual response to an initial heparin dose and enables the perfusionist to estimate the amount of additional heparin needed to achieve a certain target. The STATS block 112 maintains a historic database and enables to generate a wide variety of statistics for selected patient populations. At least the preoperative information block 101, the Ht-Hb graph generator 110, the heparin dose response curve 111 and the timers module 106 interface with the graphical user interface or GUI module 113 as is indicated by the arrows 190, 188, 187 and 186 in Fig. 1. The GUI module 113 generates the various GUI screens for interfacing with the user. Various of these GUI screens will be described in the following paragraphs. The interfacing with the user may be through a monitor in the operation room, preferably a touch screen based monitor, or may be remote via an application running on the user's smartphone, tablet PC or laptop, as is indicated by the ECMO REMOTE block 115 that transfers the screens generated by GUI 113 to the remote application as is indicated by arrow 189. In particular for ECMO, a simplified form of CPB, remote monitoring of the life -support is advantageous since it saves the medical personnel in the hospital from doing so.

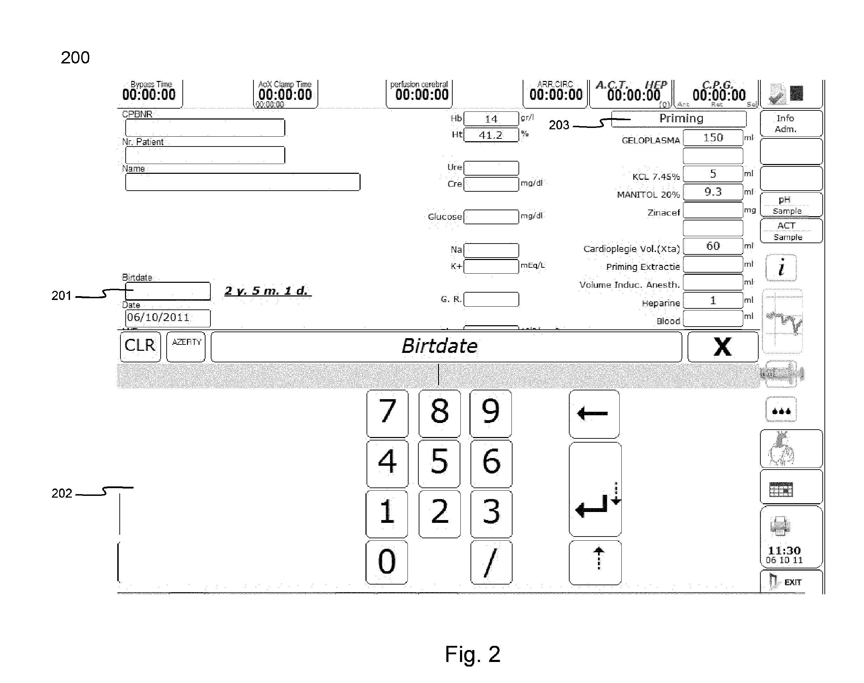

Fig. 2 shows a GUI screen generated by GUI 113 to collect preoperative information for module 101 related to the patient. The input fields that are shown on the screen are chosen and configured in advance by the user via configuration module 114. The visible input fields, their labelling and the position on the screen can be updated via the configuration module 114. By clicking on one of the input fields 201, keyboard 202 appears in the screen enabling the user to enter the preoperative data for that field. The keyboard that appears automatically adapts to the type of information that is requested, e.g. alphanumeric information such as the name, numeric information such as the postal code, date information such as the birth date, a list of clickable options such as the gender, blood group, etc., and/or a list of possible answers that is pre-entered by the user during initialisation.

After entering the preoperative patient information 121, the perfusionist can select the composition of the priming from a number of priming compositions that were are entered and named beforehand during initialisation. The pre-programmed priming 203 shall than appear on the preoperative data screen 200. To complete the entering of preoperative patient data, the perfusionist must specify the volume of priming fluid extracted before the start of the CPB, the expected volume of crystalloid cardioplegia, the eventual volume of blood withdrawn from the patient before starting the CPB, and the volume used for inducing anaesthesia. This is not shown in Fig. 2.

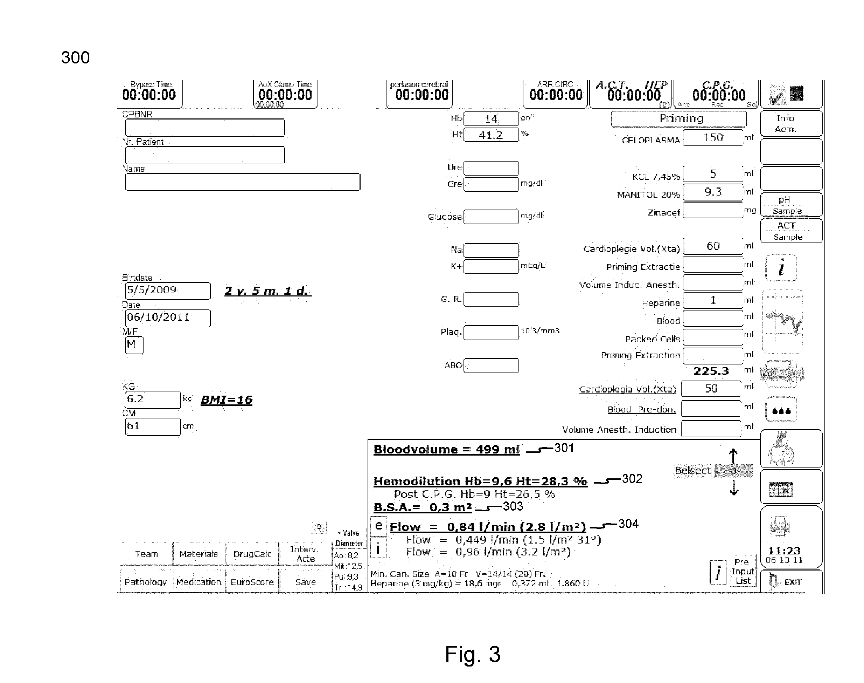

Fig. 3 shows a GUI screen 300 generated by GUI 113 with input from the preoperative calculation module 102. Starting from the preoperative patient data 121, a number of calculations are made by the preoperative calculations module 102, like for instance the body surface area or BSA 131 or 303, the blood volume 132 or 301, and the theoretical weight, T_WEIGHT or 133. Other parameters that may be calculated by the preoperative calculation module 102 are the total volume of priming fluid required, the age of the patient, the body mass index or BMI of the patient, the expected hemodilution 302, the expected hemodilution after cardioplegia, the theoretical flow rate 304 for different index values, the normalised weight, the normalised body surface area, the normalised flow rate, and the initial heparin dose in mg. For paediatric CPB, the preoperative calculation module 102 or the priming module 103 may further determine the expected valve diameters 164 and the expected cannula sizes to be used.

The theoretical weight 133 for adults is for instance calculated as follows:

- for male persons: 50 + (2.3 x ((L / 2.54) - 60))
- for female persons: 45.5 + (2.3 x ((L / 2.54) - 60))

with L being the patient's length expressed in cm.

The blood volume is for instance calculated as follows:

- for patients older than 14 years, the formula known from Smetannikov Y, Hopkins D., described in "Interoperative Bleeding: A Mathematical Model for Minimizing Hemoglobin Loss", and published in Transfusion 1996; 36: 832; and
- for patients up to 14 years, the formula known from Linderkamp O., Versmold HT. et al., described in "Estimation and Prediction of Blood Volume in Infants and Children", and published in Eur J. Pediatr 1977; 125; 227-234.

The body surface area or BSA 131 may be calculated according to different methods respectively described by Dubois D, Dubois EF. in Arch. Intern. Med.

1916; 17:863-871, by Gehan EA, George SL. in Cancer Chemother. Rep. 1970; 54: 225-235, or by Mosteller RD. N. in Engl. J. Med. 1987; 317: 1098. To adjust the method for calculating the BSA, the word "B.S.A." is clicked in the screen 300. The user can then select the desired formula 304 for BSA calculation. In order to modify the index for calculation of the flow rate, the user can click on the word "Flow" in screen 300 and select the desired index, e.g. l/m$^2$.

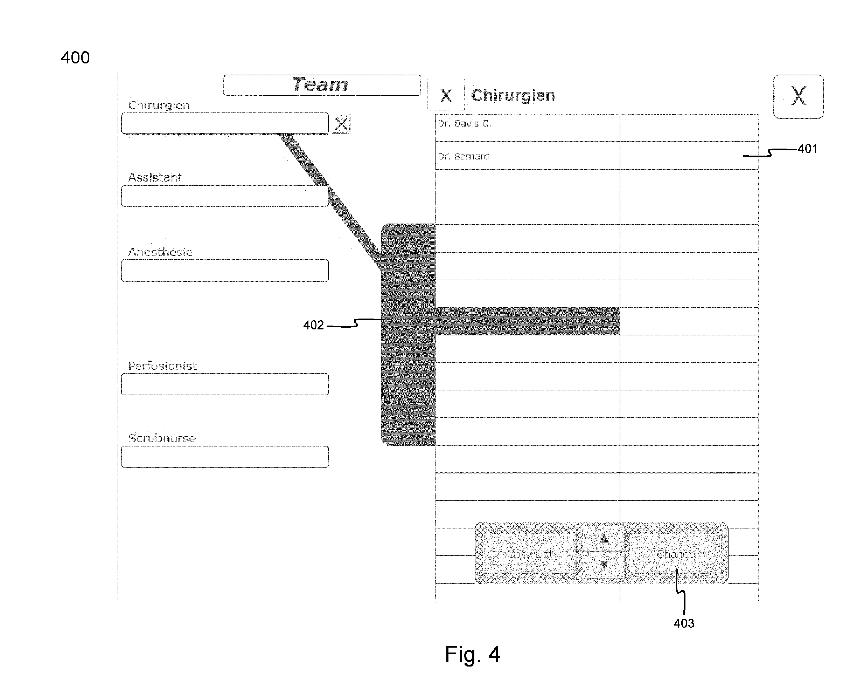

Fig. 4 shows a GUI screen 400 generated by the GUI module 113 to collect team information for the preoperative information module 101. The team information contains the names of the medical staff that will assist during the CPB, i.e. the names of the surgeon, assistant, anaesthesiologist, perfusionist, cardiologist, nurse, etc. The fields shown on this screen 400, their labelling and their positioning is in advance configured by the user through configuration module 114. By clicking on one of the fields, a list of pre-entered team member names appears. The pre-entered names are configured at initialisation using the configuration module 114. One of the names in the list can be selected and will be copied into the corresponding field after confirmation via the "enter" button 402. Alternatively, the "change" button 403 can be used to change a name or enter a new name of a team member. Thereto, an alphanumeric keyboard will be displayed as soon as the "change" button 403 is clicked.

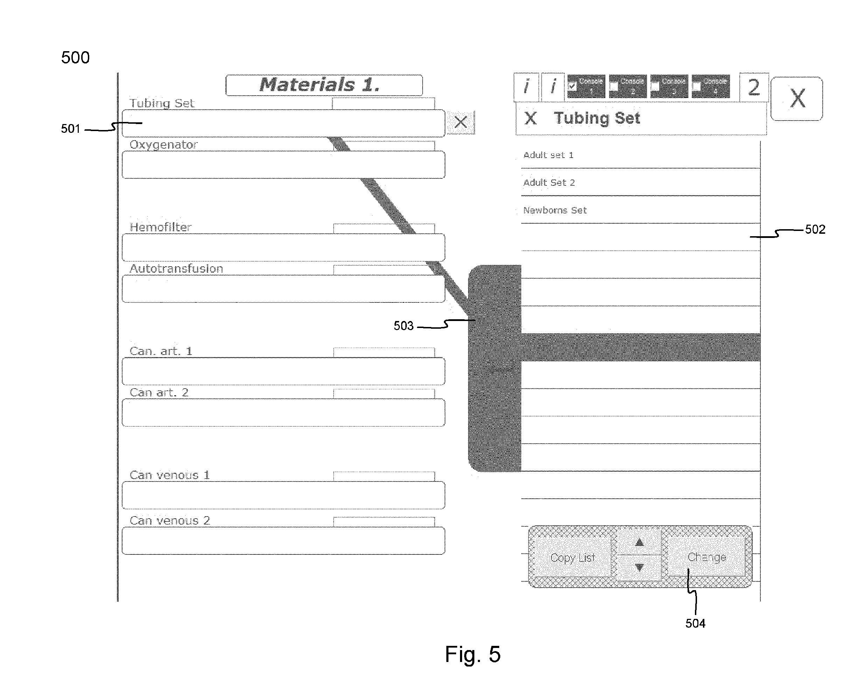

Fig. 5 shows a GUI screen 500 that is generated by the GUI module 113 to collect information on the materials 125 used during CPB, such as an identification of the set, oxygenator, cannula, etc. The fields shown in screen 500 are preconfigured by the user during initialisation via the configuration module 114. When entering the material information 125, the user can select items from a list of materials that is also pre-entered via the configuration module 114. When clicking on a field 501 in screen 500, the list of pre-entered materials pops-up. An item can be copied into the corresponding field by selecting the item and touching the "enter" button 503. Though the "change" button 504, an item can be changed or a new item can be entered and added to the list maintained for that particular field. When clicking the "change" button, a keyboard appears in screen 500 enabling the user to modify or enter an item. It is noticed that the fields for collecting the material information 125 may be spread over multiple screens or pages between which the user can easily swap.

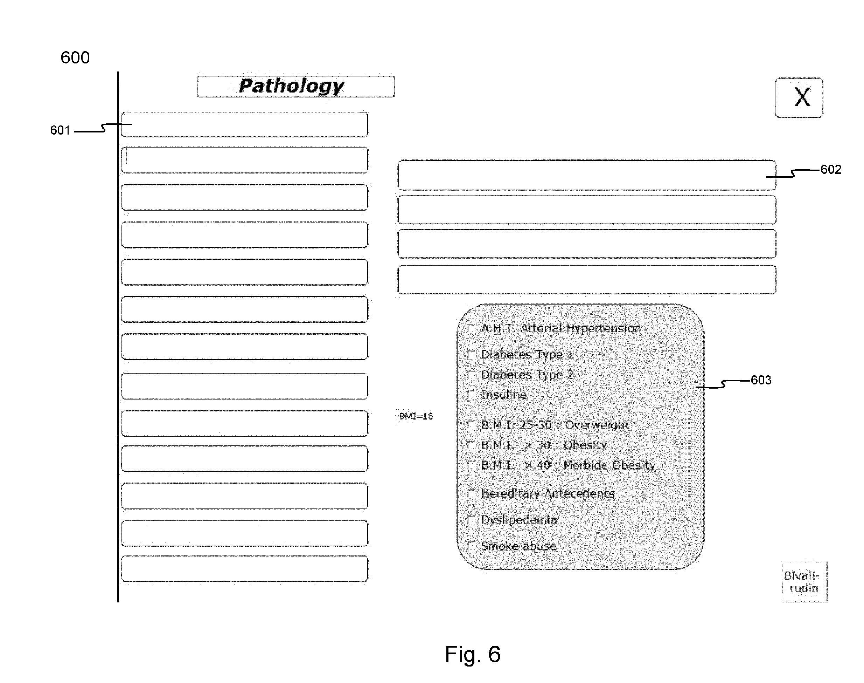

Fig. 6 shows a GUI screen 600 with input fields 601 for pathology information 122. If any one of the fields is clicked, the same list 603 of pathologies will appear. By selecting an item, the item of the list 603 gets copied to one of the fields 601. It is possible to add additional information in relation to the pathology in the input fields 602.

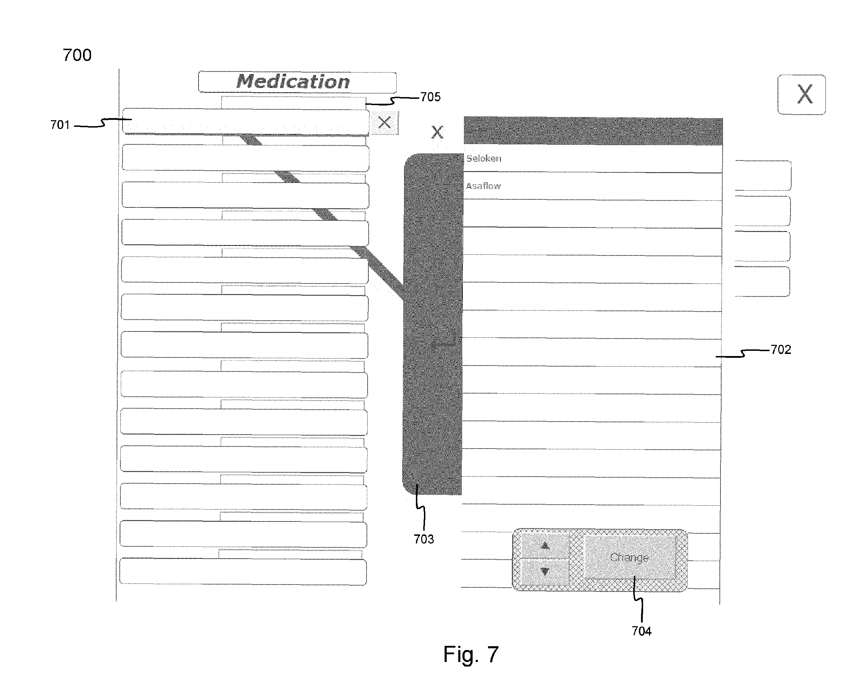

The GUI screen 700 depicted in Fig. 7 is generated by the GUI module 113 to collect medication information 123 for the preoperative data module 101.

When clicking one of the input fields 701, the same list of drugs 702 shall appear, allowing the user to select a drug and copy it into one of the fields 701 via the "enter" button 703. The "change" button 704 again enables the user to modify an item or enter a new item in the drug list 702. Each filed 701 further has a black frame 705. When the black frame 705 is clicked, a keyboard shall appear enabling the user to enter the medication dose, e.g. "3 x 2,5 mg/day".

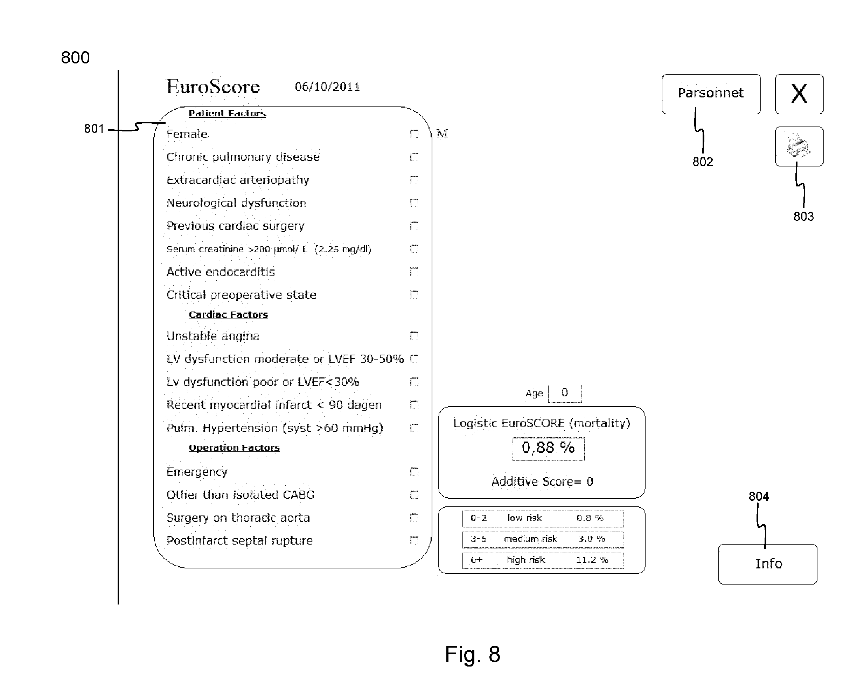

Fig. 8 shows a GUI screen 800 that is generated by the GUI module 114 using information received from the operation risk calculator 105. In order to calculate the Euroscore operation risk 141, the user must indicate in screen 800 which parameters 801 are applicable to the patient. In order to calculate the Parsonnet operation risk 142, a similar screen can be opened by touching the "Parsonnet" button 802. The operation risk calculation, i.e. the mortality calculated according to Euroscore or Parsonnet formulae, can be printed via button 803. Information on either the Euroscore or Parsonnet formulae is accessible through the "information" button 804.

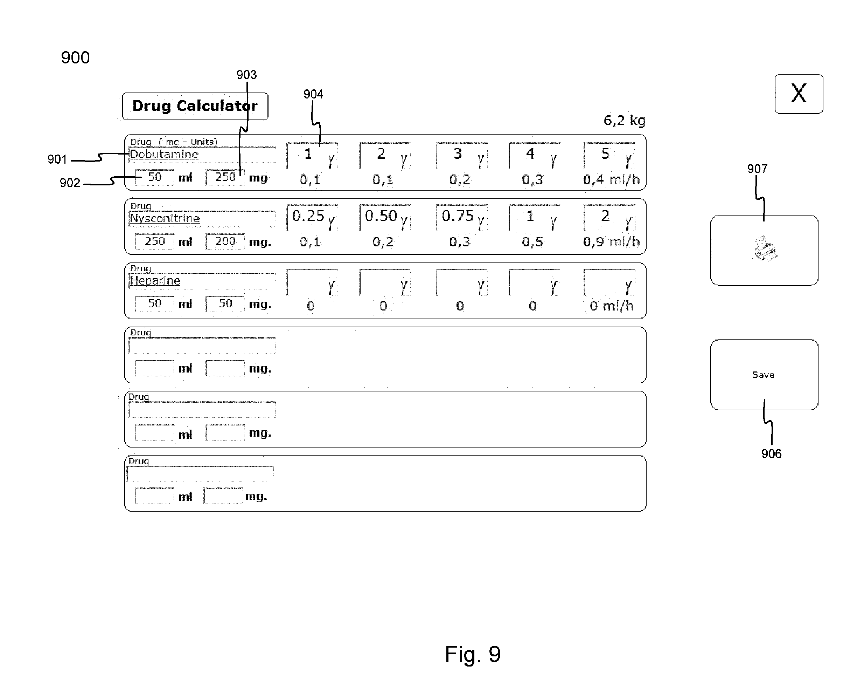

Fig. 9 shows the GUI screen 900 that is generated by GUI module 113 with information produced by the drug calculation module 104. This drug calculation module 104 calculates the medical dose 151 that must be administered during operation, i.e. how many ml/hour must be administered of a specific solution with a specific amount of active substance (in mg), in a well-defined total amount of liquid (in ml) in order to achieve a dosage expressed in gamma. For each drug, the screen 900 contains a field for the drug name 901, a field 902 to express the total quantity in ml, a field 903 to express the total quantity of active substance in mg, and five fields 904 for possible quantities for five different doses. All changes made in this screen 900 are temporarily until the user touches the "save" button 906. The calculated drug doses can be printed using the "print" button 907.

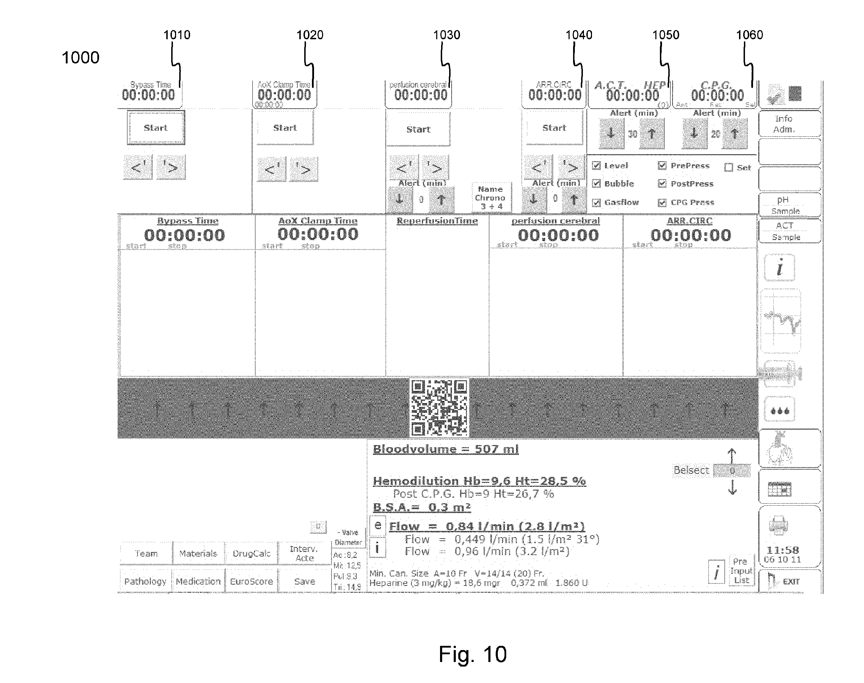

Fig. 10 illustrates a GUI screen 1000 that is generated by the GUI module 113 on instruction of the timers module 106. The GUI screen 1000 contains six chronometers: a first timer 1010 for measuring the bypass time, a second timer 1020 for measuring the aorta clamp time, a third timer 1030 and a fourth timer 1040 that are user configurable through configuration module 114, a fifth timer 1050 to measure the time lapsed since the last ACT, and a sixth timer 1060 for measuring the time lapsed since the last CPG dose. It is noticed that when the aorta clamp time chronometer 1020 is stopped while the bypass time chronometer 1010 is not yet stopped, the recirculation time can be seen. By clicking in a chronometer in screen 1000, the chronometer menu will open. This chronometer menu will enable to start the chronometer, start the chronometer one minute or click earlier than the actual time, or start the chronometer 1 minute or click later than the actual time. The chronometer menu also allows the user to stop the chronometers. For each of the timers, the total time since starting the chronometer can be seen in the middle whereas the interval time - in case there are for instance multiple clamp times - is displayed in smaller fonts. By clicking in the blank field near each chronometer in screen 1000, the times menu will open. The times menu displays all start times, stop times and total times of all chronometers. The labelling of the third timer 1030 and fourth timer 1040 can be modified via the configuration module 114. Start and stop times of the third and fourth chronometers 1030 and 1040 are then saved in a database under the user-defined name (s) for these chronometers. The fifth timer 1050 starts automatically after entering a new ACT value. By clicking on the space in screen 1000 that is occupied by the fifth chronometer 1050, a window opens that allows the user to enter the ACT value and heparin dose. The new ACT value or the new heparin dose is saved, using the actual time or an earlier or later time when entered so by the user. In addition, the user can specify the length in time that an audible and/or visual alarm must be generated to warn the perfusionist with respect to ACT. The last measured ACT value is always displayed as part of the timer 1050. Similarly, the sixth timer 1060 starts automatically after entering a new CPG amount. When clicking on the space in GUI screen 1000 occupied by the sixth chronometer 1060, a window opens that allows the user to enter the CPG doses. The user can enter the anterograde CPG, the retrograde CPG and the selective CPG. The new CPG doses are saved with the actual time unless the user specifies an earlier or later time to be saved with the new CPG doses. The user can further specify the length in time that an audible and/or visual alarm must be generated to warn the perfusionist with respect to CPG. The total amount of CPG is always displayed as part of the timer 1060. In order to enable the CPB monitoring tool to correctly calculate the effect of the CPG on the hemodilution, the user can indicate which percentage of the CPG that is not blood. The part that is not blood can be taken into account to indicate the effect of the CPG on the haemoglobin and haematocrit. It is further noticed that the chronometer menu for the third, fourth, fifth and sixth timer, contain timer alerts that can be set on or off.

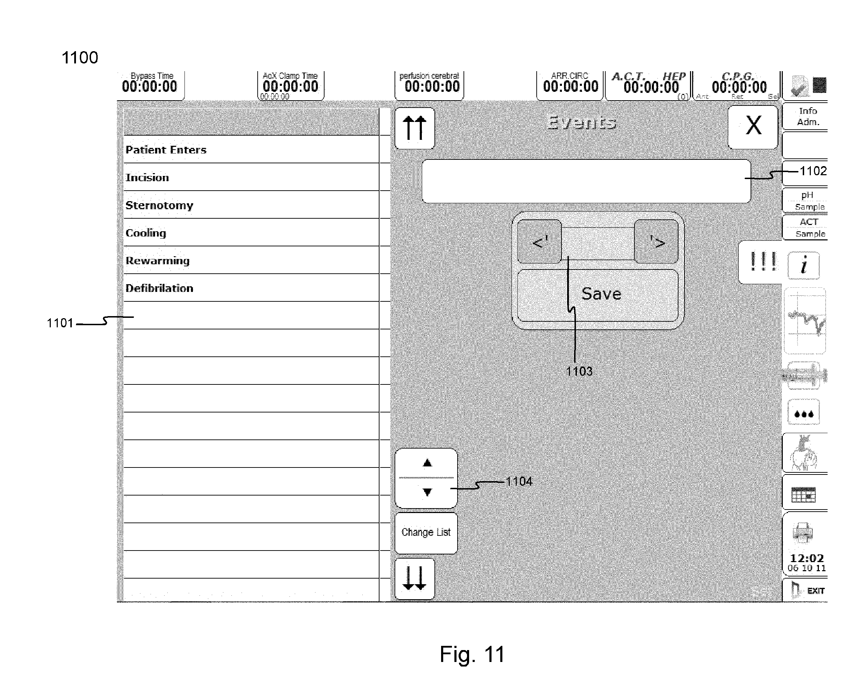

Fig. 11 shows a GUI screen 1100 that is generated by the GUI module 113 to collect information on events during CPB for the events module 108. A list of events 1101 is available. These events can be selected to become copied in the events entry field 1102. The information can be saved with the actual time or the time may be modified using the buttons 1103. To change the sequence of lines, the buttons 1104 are used. When clicking on a line in the list 1101, the information in the list can be modified or a new event can be added to the list. A keyboard will appear in the screen 1100 to assist the user in changing or adding new events to the list 1101.

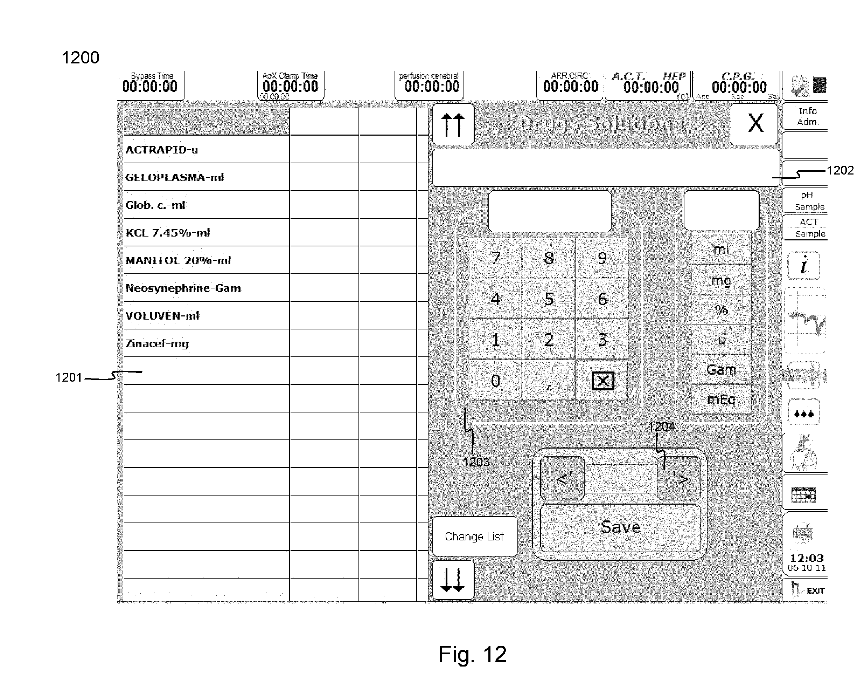

Fig. 12 shows a GUI screen 1200 that is generated by the GUI module 113 in order to collect infor on medication and solutions that are administered during the CPB procedure. A list 1201 appears in this screen 1200. An item in the list 1201 can be selected and copied into the medication entry field 1202. The numerical keyboard enables the user to enter the quantity of the drug or solution that is administered, and to select the unit wherein the quantity is expressed. The information is saved in the database with the actual time unless the user specifies an earlier or later time via the buttons 1204. When clicking on an item in the list 1201 or clicking on a blank line in the list 1201, the item can be modified or a new item can be added to the list 1201. A modification screen will appear enabling the user to change the name, packaging or volume per package. Alternatively, a keyboard will appear enabling the user to enter information with respect to a new drug or solution.

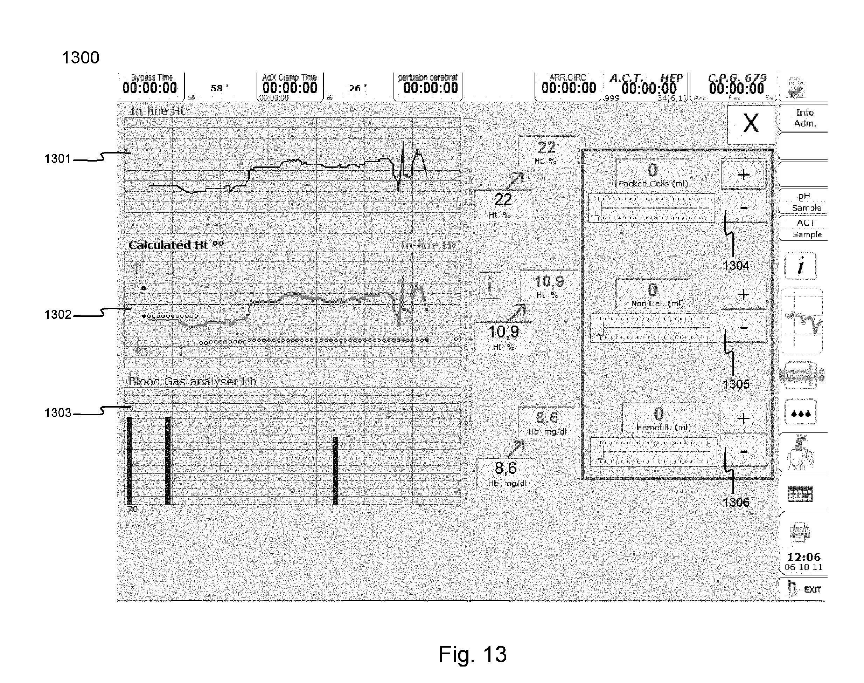

An advantageous feature of the CPB monitoring tool according to the invention is illustrated by Fig. 13. This GUI screen 1300 displays the evolution of the patient's hemodilution in order to give the perfusionist a better view on the evolution of the oxygen transport capacity of the circulating blood. The screen 1300 thereto contains three graphs that follow different parameters: graph 1301 depicts the evolution of the in-line haematocrit, graph 1302 depicts the evolution of the calculated haematrocrit, and graph 1303 depicts the evolution of the haemoglobin values measured via gasometry. Whereas the first and third graphs, 1301 and 1303, show values that are measured and stored in a database, the second graph 1302 shows the evolution of the theoretically calculated haematocrit. This calculation done by the Ht-Hb graph generating module 110 is based on the patient's haematocrit measured before the CPB, the patient's calculated blood volume based on length, weight, gender and age, the priming, the solutions and medication administered during CPB, and the diuresis and hemofiltration. The patient's calculated blood volume is not always perfect. A number of pathological conditions, medication or the patient's overall condition may influence the accurateness of this parameter. The calculation should therefore be checked by a lab test and correction of this parameter in the CPB monitoring tool according to the invention is foreseen. In order to verify if the theoretical calculation of the blood volume is acceptable or not, the difference between the calculated haematocrit and the haematocrit measured at the beginning of the CPB procedure is examined. In case there is a significant difference between these two values, the predicted blood volume must be changed accordingly. This can be done via the screen enabling entry of preoperative data. As soon as the blood volume is modified there to match the laboratory value at the beginning of the CPB, all calculations with respect to hemodilution will be correct. The changes to the calculated haematocrit can be followed via the graphs displayed in GUI screen 1300. The actual effect on the hemodilution of for instance addition of solutions during the CPB, or the effect of for instance hemofiltration, etc. can be followed, enabling the perfusionist to take better founded decisions based on up-to-date information. The "+" and "-" buttons 1304, 1305 and 1306 in GUI screen 1300 allow the user to indicate the respective amounts of packed cells, non-cellular solutions and hemofiltration administered, whereas the graphs 1301, 1302 and 1303 visualize the effect on the hemodilution.

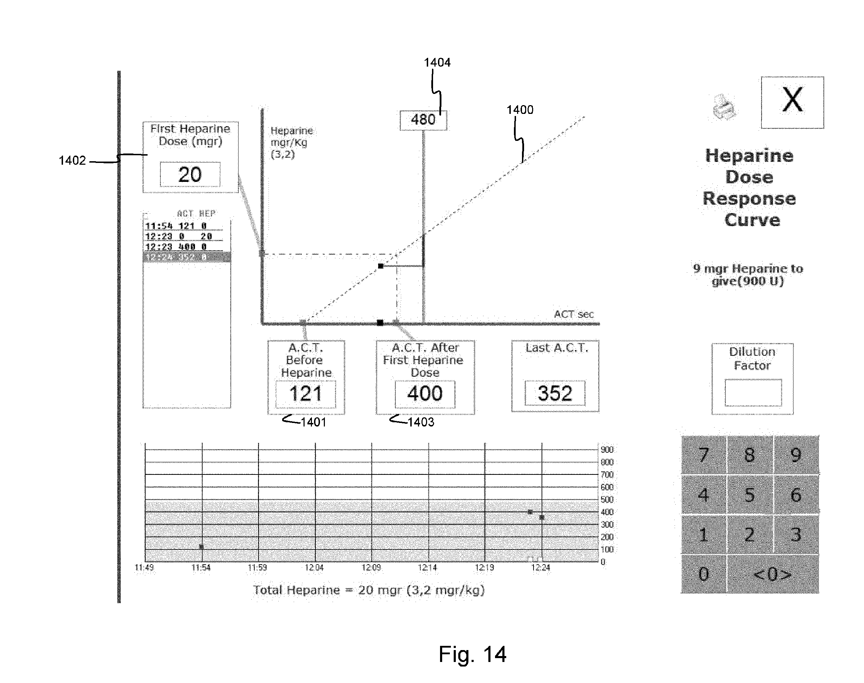

Fig. 14 shows the heparin dose response curve 1400 that represents the patient's individual reaction to a specific amount of heparin. This curve is generated by the heparin dose response curve module 111 and made accessible by GUI module 113 via the chronometer GUI screen 1000, more particularly via the fifth chronometer 1050 therein. The patient's individual reaction to heparin is used to calculate the amount of heparin that must be added to reach a specific target ACT value. Thereto, the ACT value 1401 before heparin dose, the first heparin dose 1402, and the ACT value 1403 measured after the first heparin dose must be entered chronologically in order to enable the module 111 to draw the heparin dose response curve 1400 correctly. The angle of this curve then determines the patient's individual reaction to the first heparin dose and enables to predict the amount of extra heparin that is needed to obtain a target ACT value 1404 that is specified by the user. Each time a new ACT measurement takes place, the curve 1400 is updated. If the final ACT value is smaller than the minimum desired ACT value, the CPB monitoring tool shall indicate the amount of heparin that must be added to achieve the desired ACT value. Further, the CPB monitoring tool according to the invention enables to take into account the dilution factor.

As soon as the bypass timer is stopped, the heparin dose response GUI screen shall indicate how much heparin is still active. Thereto, two calculation methods are used by module 111. According to the "120 minute half-life method", all heparin doses that were administered together with their times of administration are analysed in order to calculate the amount of heparin that is still active when the CPB procedure is stopped. This is done based on metabolization of half the heparin dose every 120 minutes. Alternatively, according to the "last measured ACT method", the last measured ACT value together with the individual heparin dose response curve are used to determine how much heparin is still active at the point in time where the last ACT value is measured.

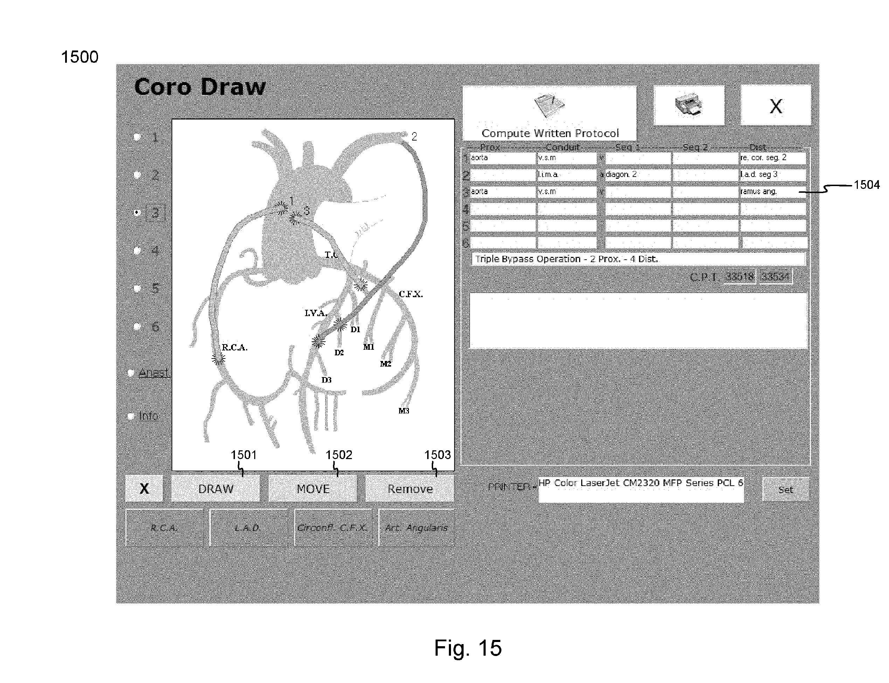

Fig. 15 shows the GUI screen 1500 generated by module 113 in collaboration with an optional draw module not shown in Fig. 1. This draw module allows the user to draw coronary bypasses. Up to six bypasses can be drawn. Thereto, the user identifies the number of the bypass, selects in screen 1500 the place where the bypass begins, identifies additional points of the bypass, and at last selects in screen 1500 the place where the bypass ends. This is possible as long as the "Draw" button 1501 is activated. The course of a previously drawn bypass can be modified using the "Move" button 1502, and the last drawn part of an active bypass can be removed using the "Erase" button 1503. When all bypasses have been drawn, the computer program will examine the drawing and display data in the fields 1504. Each bypass that begins at the aorta will receive the designation "VSM" or vena saphena. If the bypass is not a VSM but for instance a free mammary artery, the conduit field must be modified by selecting a conduit out of a list of possible conduits that appears. To indicate a sequential anastomosis, the complete bypass is drawn. Thereafter, the exact location where the sequential anastomosis should be placed, is identified. The tool further enables to enter additional, specific information with respect to the anastomosis or conduit. A menu appears that allows to indicate which thread is used.

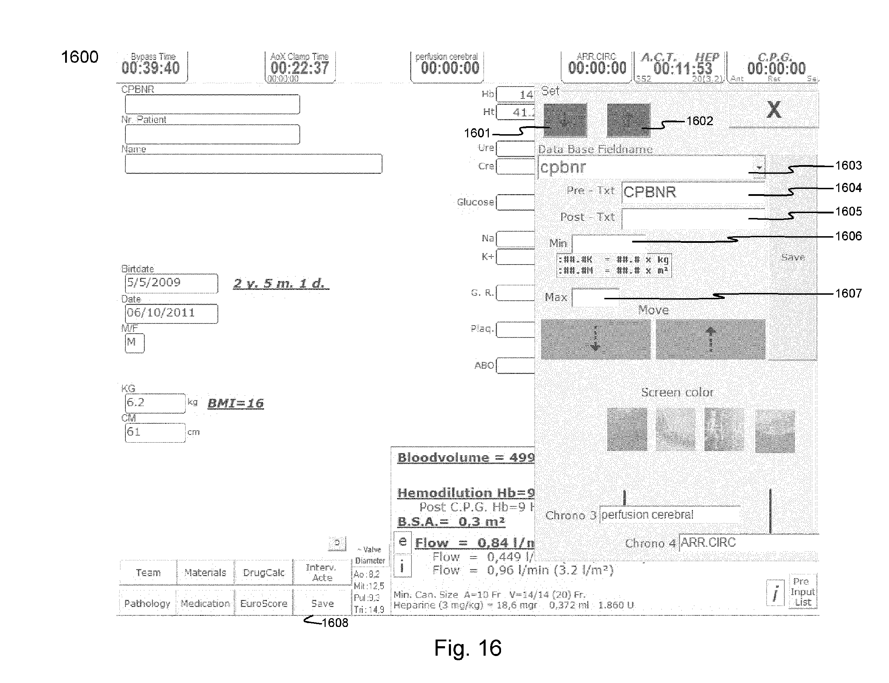

Through the configuration module 114, the preoperative data input fields can be configured: the desired parameters and their location on screen 200 can be set. This is illustrated by Fig. 16 which shows how the GUI screen 200 for preoperative data is configured. The buttons 1601 and 1602 allow to choose the location on screen 200 where the parameter entry field is positioned. The possible locations may be organised in a table, e.g. 35 locations in 2 columns. When clicking on the field 1603, the list of possible parameters for entering preoperative information becomes visible. This list may for instance contain the patient's identification number, the second patient's identification number, a CPB number, the patient's last name and first name, the date if intervention, the patient's birthdate, the patient's gender, the patient's social security institution number, the patient's social security number, the patient's address, the weight, length, blood group, haemoglobin, haematocrit, red blood cells, white blood cells, thrombocytes, total proteins, K, Na, Ca, Mg, Urea, creatinine, glucose, left ventricular ejection fraction, operating room, etc. In the filed 1604, the user can enter or change the label of the field. In the field 1605, the user can enter or change the unit wherein the parameter value must be expressed. In case the parameter is related to a list of choices, the possible choices must be entered. The "Min" and "Max" fields, 1606 and 1607, enable the user to specify the limits in case the parameter is numerical. The parameter, its labelling and positioning are confirmed through the "Save" button 1608.

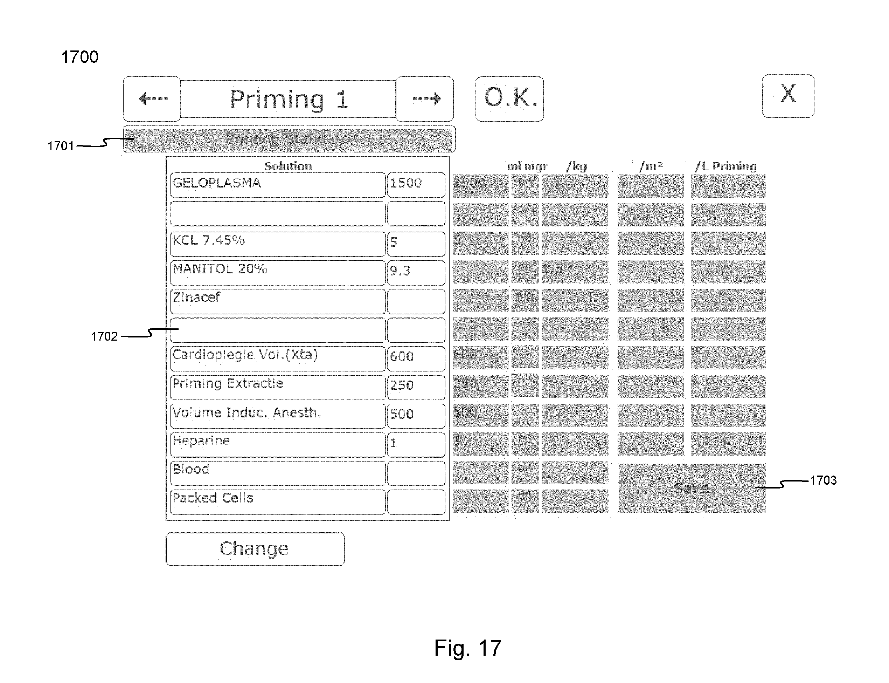

Fig. 17 illustrates configuration of the priming compositions through the configuration module 114. The priming screen 1700 enables to change or enter the pre-programmable priming compositions of for instance 10 priming fluids. The name of the priming set is entered in field 1701. Further, table 1702 allows the user to enter the constituents and their amount in various units. The amount may for instance be expressed in ml/kg patient weight, such that the amount needed for a particular patient can be exactly calculated in function of the patient's weight. Alternatively, the amount may be expressed in $ml/m^2$ BSA. This way, the amount needed for a particular patient can be exactly calculated in function of the patient's BSA. According to yet another alternative, the amount may be calculated in ml/l priming. The priming constituents can be selected from a list of solutions. The pre-programming of a priming composition is confirmed through the "Save" button 1703, as a result of which the priming composition becomes available for use.

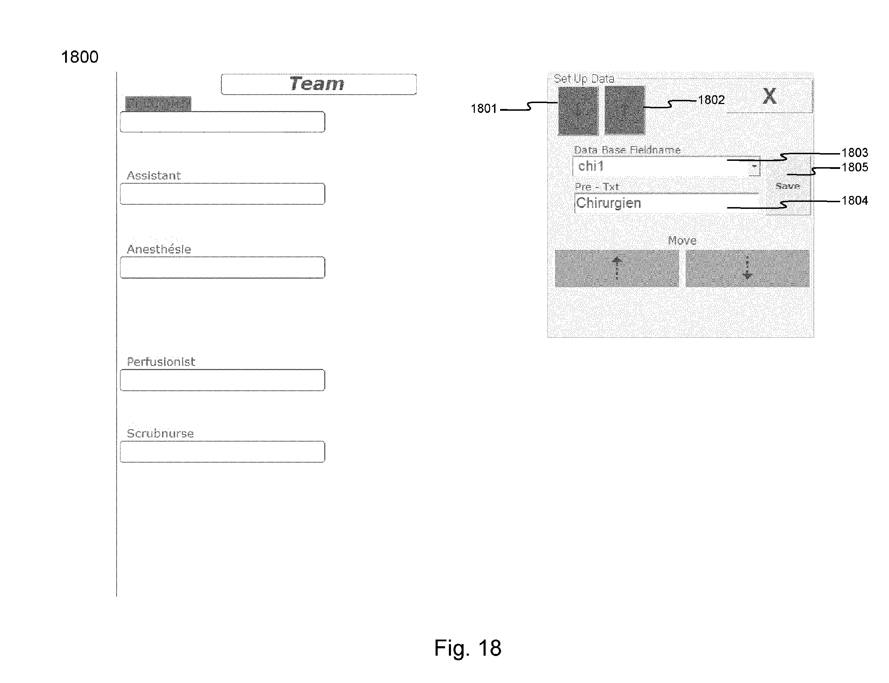

Fig. 18 illustrates initialisation of the name list of the medical team through the configuration module 114. The desired parameters, and their location in the medical team screen 400 is set-up. Thereto, the buttons 1801 and 1802 are used to determine the position of the entry field in screen 400. The desired parameter is then selected from a list that is accessible through clicking field 1803. Possible parameters for the medical team data are the names of several surgeons, the names of several anaesthesiologists, the names of several perfusionists, the names of several instrumentists, the names of several nurses, the name of the cardiologist, the names of several family doctors, etc. The field 1804 enables the user to specify the label of the parameter. Once the screen 400 for medical team data entry is configured, the screen configuration is memorized by clicking the "Save" button 1805.

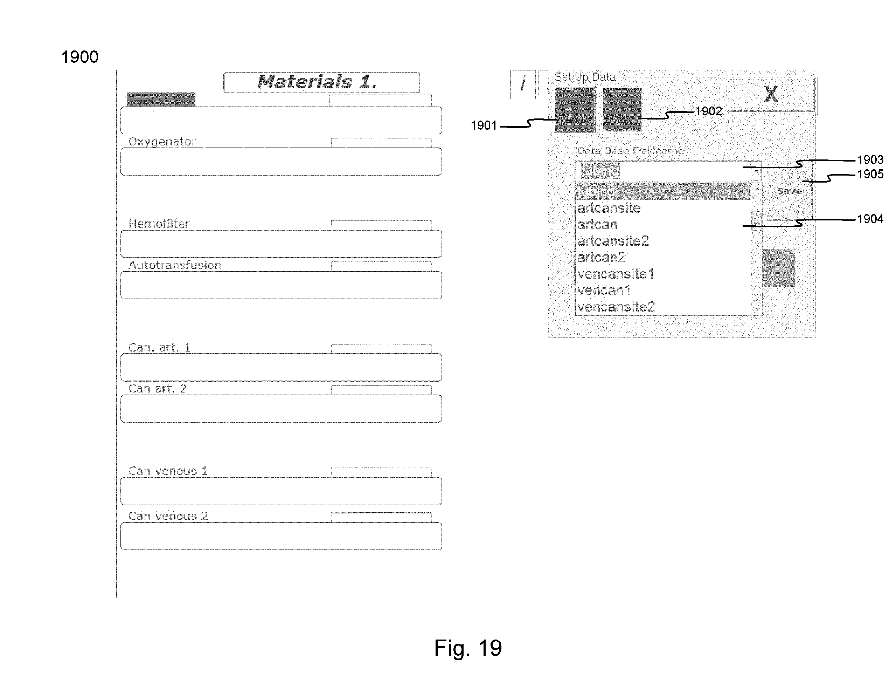

Fig. 19 illustrates initialisation of the name list for the materials through the configuration module 114. The desired parameters, and their location in material screen 500 is set-up. The arrow buttons 1901 and 1902 allow the user to select the position of the entry field in screen 500. A number of locations are available. The field 1903 gives access to a number of parameters that can be collected with respect to materials used during CPB. This list 1904 for instance may contain the name of the CPB console, the oxygenator, the venous reservoir, the cardiotomy reservoir, the hemofilter, the circuits, the arterial cannulation site 1, the arterial cannulation site 2, the arterial cannula 1, the arterial cannula 2, the venous cannulation site 1, the venous cannulation site 2, the venous cannulation site 3, the venous cannula 1, the venous cannula 2, the venous cannula 3, the cannula CPG anterograde, the cannula CPG retrograde, the arterial pump, the left aspiration cannula, the autotransfusion, etc. The user can further enter the label that has to appear near the entry field in screen 500. Once the screen 500 is configured, the "Save" button 1905 allows to memorize the configuration.

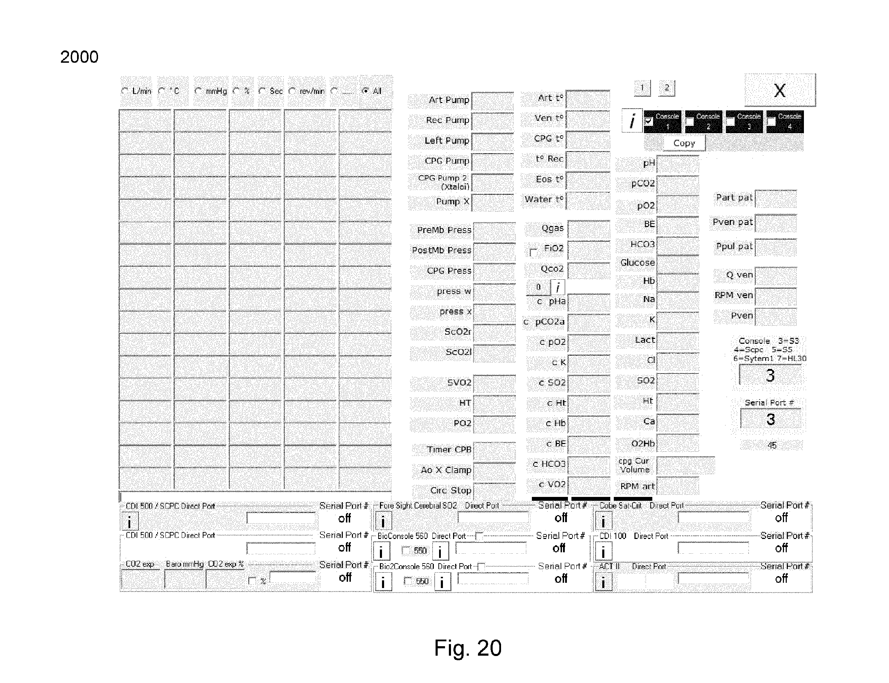

In order to adapt the CPB monitoring tool to the specific configuration of a heart-lung machine, its interface, e.g. an RS232 interface, must be configured. This is illustrated by GUI screen 2000 in Fig. 20. All the variables that are available on the specific heart-lung machine will appear: temperatures, pressures, flow rates, etc. that can be collected by the data collect module 107. In order to have the parameters appear on the screen, the serial port number for connection with the heart-lung machine must be specified correctly. To calibrate the parameters, the available parameters must be linked with the respective fields where their value should appear. The parameters available from the heart-lung machine and the fields available in the CPB monitoring tool are therefore displayed on the screen to enable the user to link them. Once this is done, the measured value of a parameter will appear in the chosen field. Other devices can be connected as well. Thereto, their serial port number must be specified.

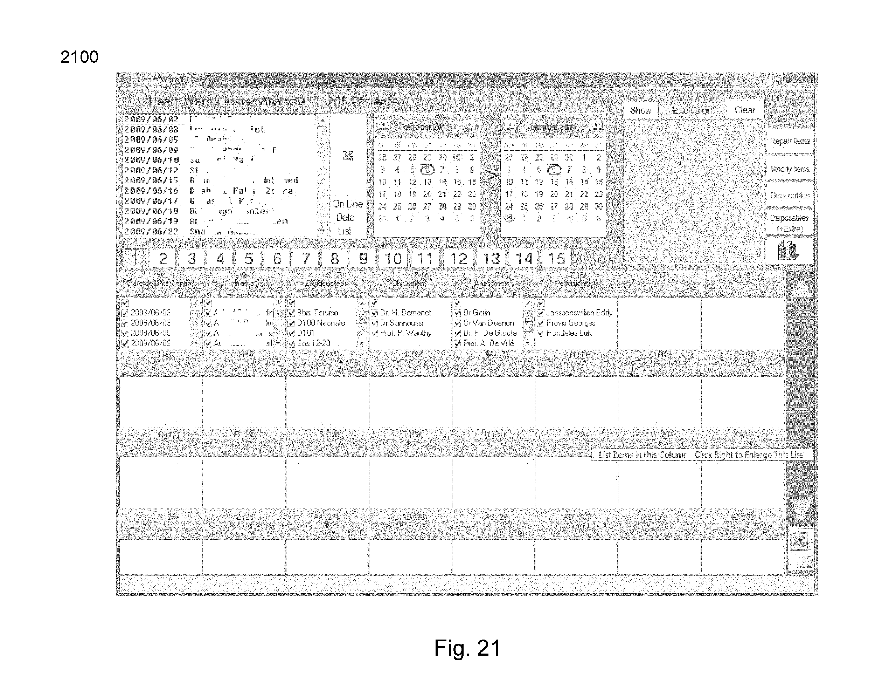

Fig. 21 shows a GUI screen 2100 generated by the statistical module 112. This statistical module 112 encompasses a number of different algorithms and methods for grouping objects in a way that the degree of association between two objects is maximal if they belong to the same group and minimal otherwise. The statistical module 112 in other words discovers structures in data. The statistics module 112 lists the parameters intuitively and allows the user to include/exclude criteria in different parameters. The data are then intelligently clustered in data groups and graphic representations of the distinguished data groups are generated. All parameters stored during the CPB procedure with respect to one patient can be collected, exported, stored and printed for further analysis. A listing of all patients is generated, and each of the columns in the list can be populated with one of the parameters available in the database. Once the list is made up, it is kept in memory. Thereafter, exclusion/ inclusion criteria are defined, e.g. the starting and ending date of the list, patients with a particular blood group, gender, etc. Once the inclusion/exclusion criteria are defined, the list can be exported. Also exported are the number of patients, the period in time, the complete set of exclusion criteria and a graphical representation of the column's parameter appearance. These graphics can be copied into another application, e.g. Powerpoint. Through a repair function, the statistical module 112 repairs the data when new patients are added. Further, the repeatability is improved through a modify function in the statistical module 112 that deals with spelling, word order, the use of capitals, etc. and avoids that such kind of errors have an influence on the statistics. Several pre-programmed graphics are then available, like for instance bar graphics. The minimum and maximum values for each of the bars in these graphics are adaptable by the user, the y scale can be set automatically or by the user for minimum and maximum scan graphics, the X and Y axes can be swapped in XY graphics, etc.

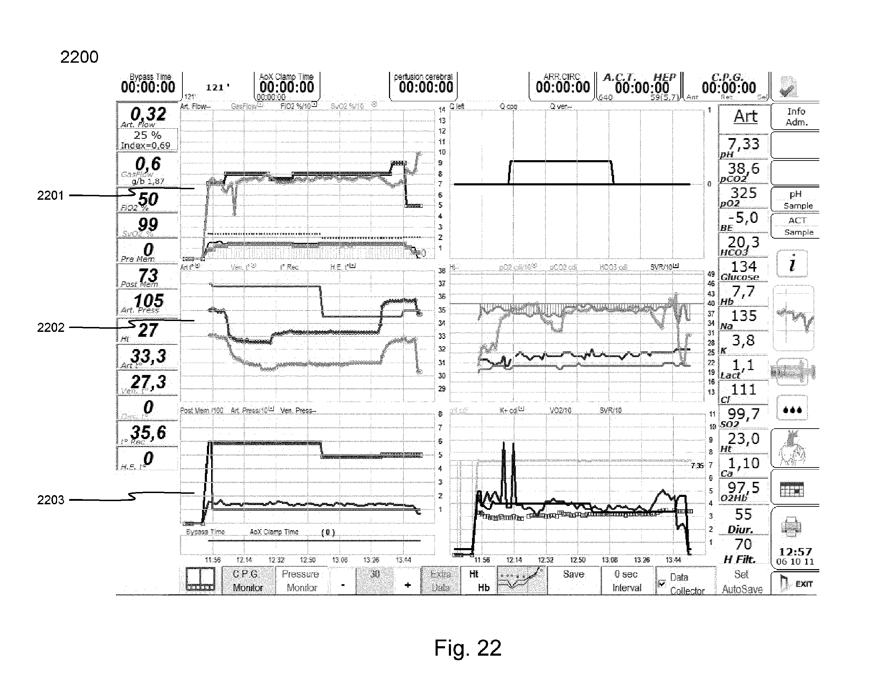

Fig. 22 shows a GUI screen 2200 representing the main working screen during the operation procedure, showing all measured and derived parameters and their evolution in time. Derived calculated parameters like oxygen consumption and systemic vascular resistance curves are continuously visualised in order to assist the perfusionist during the procedure. The absolute minimum blood flow needed to assure vital oxygen delivery is constantly calculated taking into account the temperature, hemodilution and morphology of the patient. The actual cardiac index 2201, i.e. the blood flow per $m^2$, is constantly visualised. The temperature difference 2202 between patient temperature and blood temperature is monitored and shown in order to alert the perfusionist when temperature gradients become too large. The in-line pressure differences 2203 measured before and after the membrane oxygenator are constantly shown to be evaluated by the perfusionist during the procedure and to alert him in case of overpressure. All this among other features makes the tool according to the present invention much more than just a data logger to produce a database, but a real monitor assisting the perfusionist during the procedure and enabling him to make better founded decisions throughout the whole procedure. Thanks to the tool according to the present invention, the perfusionist has a better view on the individual reactions of the patient at any moment during the procedure. This additional information helps the perfusionist to have a better quality judgement and be able to make changes in the treatment based on more reliable data.

This main screen 2200 shown during the bypass operation can be configured through the configuration module 114. The number of curve fields that is displayed can be varied, e.g. 1 to 3 left side curve fields and 1 to 3 right side curve fields. The maximum and minimum values shown along X- and Y-axes, the units, and certain aspects of the curves can be configured as well in order to increase the user-friendliness and intuitive interaction with the perfusionist during operation.

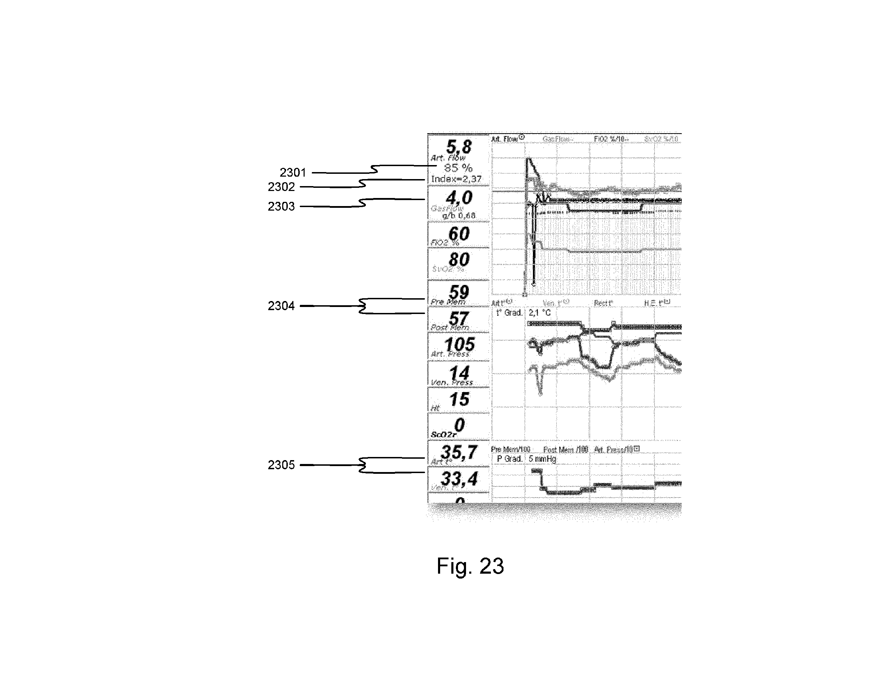

Fig. 23 illustrates visualization of derived parameters in GUI screen 2200 such as the percentage of the blood pump flow compared to the pre-calculated flow 2301, the index 2302 of the blood flow referenced to the body surface area in $L/m^2$, the ratio of gas flow versus blood flow 2303, the temperature gradient between blood and patient temperature 2304, and the pressure difference 2304 between the pre-oxygenator and post-oxygenator line pressure. These measurements are constantly visualised to inform the perfusionist at any moment during the procedure.

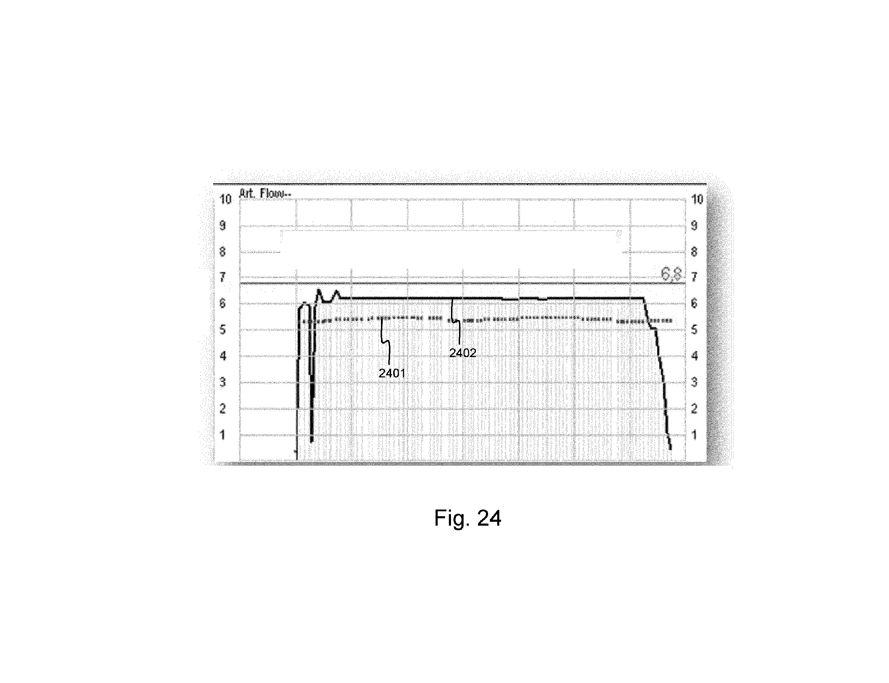

In Fig. 24, the dotted line 2401 shows the minimum blood flow needed to transport oxygen to the whole body taking into account the morphology of the actual patient, the actual temperature, and the actual hemodilution at any point in time during the operation procedure. The arterial pump flow is shown by the full line 2402.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A cardiopulmonary bypass or CPB monitoring tool (100) comprising:

   - a preoperative information module (101) enabling entry and management of patient data (121), pathology data (122), medication data (123), operation team data (124), material data (125) for use during operation;
   - a preoperative calculation module (102) able to estimate a body surface area or BSA (131), blood volume (132), and theoretical weight (133) from said patient data (121);
   - a priming module (103) able to determine priming constitution (161), volume (163) and flow (162) to achieve a hemodilution target;
   - an operation risk module (105) for calculating operation risk according to Euroscore (141) and/or Parsonnet formulae (142);
   - a drug calculation module (104) able to determine medication doses (151) that must be administered during operation;
   - a timer module (106) comprising one or more timers that can be activated during operation;
   - a data collection module (107) comprising an interface and drivers enabling data collection from a wide variety of extracorporeal pumps and oxygenators during operation;
   - an events module (108) enabling entry and management of events during operation, said events module (108) enabling retroactive manipulation of the time of an event;
   - a printing report generation module (109) with user-configurable parameter selection for at least one report;
   - a graphic user interface (113); and
   - a configuration module (114) for said graphic user interface (113), said configuration module (114) enabling selection of fields for entry of preoperative information (121, 122, 123, 124, 125), labelling of said fields selected and positioning of said fields selected in data entry screens used by said preoperative information module (101) for entry of preoperative information, enabling configuration of standard priming constitutions, enabling initialisation of medical team members, enabling initialisation of materials, enabling configuration of interfaces to a wide variety of extracorporeal pumps, and enabling configuration of chart screens displayed during operation in said graphical user interface (113).

2. A CPB monitoring tool (100) according to claim 1, wherein said priming module (103) is further adapted to determine valve diameters (164) and/or cannula sizes for paediatric CPB.

3. A CPB monitoring tool (100) according to claim 1, wherein said timer module (106) comprises:

- a first timer (BYPASS) for registering bypass time;
- a second timer (AORTA CLAMP) for registering aorta clamp time;
- a third timer (ACT) for registering time lapsed since a last Anti Coagulation Time or ACT measurement; and
- a fourth timer (CPG) for registering time lapsed since a last CPG dose.

4. A CPB monitoring tool (100) according to claim 1, wherein said timer module (106) comprises one or more user-configurable timers (USER DEFINED).

5. A CPB monitoring tool (100) according to claim 1, wherein said events module (108) stores a list of standard events that take place before, during and after a PCB.

6. A CPB monitoring tool (100) according to claim 1, further comprising:

- a medication module adapted to log medication supplied during operation.

7. A CPB monitoring tool according to claim 1, further comprising:

- a theoretical and measured haematocrit evolution graph generator enabling to monitor the evolution of the patients hemodilution throughout an operation procedure.

8. A CPB monitoring tool (100) according to claim 1, further comprising:

- a heparin dose response curve generator (110) enabling to derive the patients response to a first heparin dose and to predict additional heparin doses in order to achieve a target ACT value and to predict at the end of an operation procedure how much heparin is leftover to be neutralized in order to restore normal coagulation.

9. A CPB monitoring tool (100) according to claim 1, further comprising:

- a draw module enabling drawing a coronary bypass and sequential anastomosis.

10. A CPB monitoring tool (100) according to claim 1, wherein said material module is adapted for evidence based material selection.

11. A CPB monitoring tool (100) according to claim 1, further comprising:

- a statistical module (112) for statistic calculations on a population of patients.

12. A CPB monitoring tool (100) according to claim 1, further comprising:

- connectivity (115) to an application enabling remote monitoring during extracorporeal membrane oxygenation or ECMO.

13. A CPB monitoring tool (100) according to claim 9, further comprising:

- a module for alarm generation through e-mail or SMS.

14. A CPB monitoring tool (100) according to claim 1, further adapted to visualize during operation derived calculated parameters to assist a perfusionist.

EP 2 581 846 A1

**100**

**101**

```
PREOPERATIVE
INFO
```
| 121 PATIENT |
| 122 PATHOLOGY |
| 123 MEDICATION |
| 124 TEAM |
| 125 MATERIAL |

**181** **183** **184**

**102**

```
PREOPERATIVE
CALC
```
| 131 BSA |
| 132 BLOOD_VOL |
| 133 T_WEIGHT |

**182**

**103**

```
PRIMING
```
| 161 CONSTITUTION |
| 162 FLOW |
| 163 VOLUME |
| 164 VALVE_DIAM |

**105**

```
OPERATION RISK CALC
```
| 141 EUROSCORE |
| 142 PARSONNET |

**185**

**191**

**192**

**104**

```
DRUG CALC
```
| 151 MEDICAL DOSE |

**114**

```
CONFIG
```

**195**

**110** **111** **106** **112** **109** **194** **107** **193** **108**

| Ht-Hb GRAPH | HEPARIN DOSE RESPONSE CURVE | TIMERS | | | | | STATS | PRINTING REPORTS | DATA COLLECT | EVENTS |
|---|---|---|---|---|---|---|---|---|---|---|
| | | BYPASS | AORTA CLAMP | ACT | CPG | USER DEFINED | | | | |

**188** **187** **190** **186**

**113**

```
GUI
```

**189**

**115**

```
ECMO
REMOTE
```

Fig. 1

200

Fig. 2

EP 2 581 846 A1

300

| Bypass Time | AoX Clamp Time | perfusion cerebral | ARR.CIRC | A.C.T. HEP | C.P.G. |
|---|---|---|---|---|---|
| 00:00:00 | 00:00:00 | 00:00:00 | 00:00:00 | 00:00:00 | 00:00:00 |
| | 00:00:00 | | | (0) Arc Ret Sel |

CPBNR

Nr. Patient

Name

Birtdate
5/5/2009    *2 y. 5 m. 1 d.*
Date
06/10/2011
M/F
M

KG
6.2  kg  *BMI=16*
CM
61  cm

Hb  14  gr/l
Ht  41.2  %

Ure
Cre  mg/dl

Glucose  mg/dl

Na
K+  mEq/L

G. R.

Plaq.  10'3/mm3

ABO

Priming

GELOPLASMA  150  ml

KCL 7,45%  5  ml
MANITOL 20%  9.3  ml
Zinacef  mg

Cardioplegie Vol.(Xta)  60  ml
Priming Extractie  ml
Volume Induc. Anesth.  ml
Heparine  1  ml
Blood  ml
Packed Cells  ml
Priming Extraction  ml
**225.3**  ml
Cardioplegia Vol.(Xta)  50  ml
Blood Pre-don.  ml
Volume Anesth. Induction  ml

Info Adm.

pH Sample

ACT Sample

*i*

Belsect  0

**Bloodvolume = 499 ml** —301

**Hemodilution Hb=9,6 Ht=28,3 %** —302
Post C.P.G. Hb=9 Ht=26,5 %
**B.S.A.=  0,3 m²** —303

e **Flow = 0,84 l/min (2.8 l/m²)** —304
i  Flow = 0,449 l/min (1.5 l/m² 31°)
i  Flow = 0,96 l/min (3.2 l/m²)

Min. Can. Size  A=10 Fr  V=14/14 (20) Fr.
Heparine (3 mg/kg) = 18,6 mgr  0,372 ml  1.860 U

~ Valve Diameter
Ao :8,2
Mit :12,5
Pul :9,3
Tri :14,9

| Team | Materials | DrugCalc | Interv. Acte |
|---|---|---|---|
| Pathology | Medication | EuroScore | Save |

Pre Input List

11:23
06 10 11

EXIT

Fig. 3

400

Team

Chirurgien

Assistant

Anesthésie

Perfusionist

Scrubnurse

X  Chirurgien                                    X

| Dr. Davis G. | |
|---|---|
| Dr. Barnard | |
| | |
| | |
| | |
| | |
| | |
| | |
| | |
| | |
| | |
| | |

401

402

| Copy List | ▲ ▼ | Change |
|---|---|---|

403

Fig. 4

EP 2 581 846 A1

Fig. 5

600

**Pathology**

X

601

602

BMI=16

603

☐ A.H.T. Arterial Hypertension

☐ Diabetes Type 1

☐ Diabetes Type 2

☐ Insuline

☐ B.M.I. 25-30 : Overweight

☐ B.M.I. > 30 : Obesity

☐ B.M.I. > 40 : Morbide Obesity

☐ Hereditary Antecedents

☐ Dyslipedemia

☐ Smoke abuse

Bivali-rudin

Fig. 6

Fig. 7

EP 2 581 846 A1

EuroScore    06/10/2011

**Patient Factors**

Female                                             ☐  M

Chronic pulmonary disease                          ☐

Extracardiac arteriopathy                          ☐

Neurological dysfunction                           ☐

Previous cardiac surgery                           ☐

Serum creatinine >200 μmol/ L  (2.25 mg/dl)        ☐

Active endocarditis                                ☐

Critical preoperative state                        ☐

**Cardiac Factors**

Unstable angina                                    ☐

LV dysfunction moderate or LVEF 30-50%             ☐

Lv dysfunction poor or LVEF<30%                    ☐

Recent myocardial infarct < 90 dagen               ☐

Pulm. Hypertension (syst >60 mmHg)                 ☐

**Operation Factors**

Emergency                                          ☐

Other than isolated CABG                           ☐

Surgery on thoracic aorta                          ☐

Postinfarct septal rupture                         ☐

801

Parsonnet    X

802

803

Age    0

Logistic EuroSCORE (mortality)

0,88 %

Additive Score= 0

| 0-2 | low risk | 0.8 % |
|-----|----------|-------|
| 3-5 | medium risk | 3.0 % |
| 6+ | high risk | 11.2 % |

804

Info

Fig. 8

900

## Drug Calculator

6,2 kg

Drug ( mg - Units)
Dobutamine
50 ml   250 mg

| 1 γ | 2 γ | 3 γ | 4 γ | 5 γ |
|-----|-----|-----|-----|-----|
| 0,1 | 0,1 | 0,2 | 0,3 | 0,4 ml/h |

Drug
Nysconitrine
250 ml   200 mg.

| 0.25 γ | 0.50 γ | 0.75 γ | 1 γ | 2 γ |
|--------|--------|--------|-----|-----|
| 0,1 | 0,2 | 0,3 | 0,5 | 0,9 ml/h |

Drug
Heparine
50 ml   50 mg.

| γ | γ | γ | γ | γ |
|---|---|---|---|---|
| 0 | 0 | 0 | 0 | 0 ml/h |

Drug
ml   mg.

Drug
ml   mg.

Drug
ml   mg.

901
902
903
904

X

907

Save

906

EP 2 581 846 A1

Fig. 9

Fig. 10

1100

| Bypass Time | AoX Clamp Time | perfusion cerebral | ARR.CIRC | A.C.T. | HEP | C.P.G. |
|---|---|---|---|---|---|---|
| 00:00:00 | 00:00:00 | 00:00:00 | 00:00:00 | 00:00:00 | | 00:00:00 |

Fig. 11

1200

Fig. 12

1300

Fig. 13

EP 2 581 846 A1

Fig. 14

1500

Fig. 15

EP 2 581 846 A1

1600

Bypass Time
00:39:40

AoX Clamp Time
00:22:37
(00:00:00)

perfusion cerebral
00:00:00

ARR.CIRC
00:00:00
352

A.C.T.  HEP
00:11:53
20(3.2)

C.P.G.
00:00:00
Ant  Ret  Sa.

CPBNR

Nr. Patient

Name

Birtdate
5/5/2009        *2 y. 5 m. 1 d.*

Date
06/10/2011

M/F
M

KG
6.2   kg   *BMI=16*

CM
61    cm

Hb  14
Ht  41.

Ure
Cre

Glucose

Na
K+

G. R.

Plaq.

ABO

Set

1601        1602

X

Data Base Fieldname

cpbnr                        ▼        1603

Pre - Txt   CPBNR                    1604

Post - Txt                           1605

Min                                  1606

:##.#K  =  ##.# x kg
:##.#M  =  ##.# x m²

Save

Max                                  1607

Move
↓        ↑

Screen color

Bloodvolume = 499

Hemodilution Hb=9
   Post C.P.G. Hb=9
B.S.A.= 0,3 m²

e  Flow = 0,84 l/n
   Flow = 0,449 l/
i  Flow = 0,96 l/min (3.2 l/m²)

Chrono 3  perfusion cerebral

Chrono 4  ARR.CIRC

~Valve
Diameter
Ao:8,2
Mit:12,5
Pul:9,0
Tri:14,9

| Team | Materials | DrugCalc | Interv. Acte |
| Pathology | Medication | EuroScore | Save |

1608

Min. Can. Size  A=10 Fr  V=14/14 (20) Fr.
Heparine (3 mg/kg) = 18,6 mgr  0,372 ml  1.860 U

i  Pre Input List

Fig. 16

29

1700

| ← ⋯ | Priming 1 | ⋯ → | O.K. | | X |

1701 — Priming Standard

| Solution | | ml mgr | /kg | /m² | /L Priming |
|---|---|---|---|---|---|
| GELOPLASMA | 1500 | 1500 ml | | | |
| | | | | | |
| KCL 7.45% | 5 | 5 ml | | | |
| MANITOL 20% | 9.3 | ml 1.5 | | | |
| Zinacef | | mg | | | |
| | | | | | |
| Cardioplegie Vol.(Xta) | 600 | 600 | | | |
| Priming Extractie | 250 | 250 ml | | | |
| Volume Induc. Anesth. | 500 | 500 | | | |
| Heparine | 1 | 1 ml | | | |
| Blood | | ml | | | |
| Packed Cells | | ml | | | |

1702

Save — 1703

Change

Fig. 17

1800

*Team*

Assistant

Anesthésie

Perfusionist

Scrubnurse

Set Up Data

1801 —

— 1802

X

Data Base Fieldname — 1803

chi1 — 1805

Pre - Txt        Save

Chirurgien — 1804

Move

Fig. 18

1900

## *Materials 1.*

Oxygenator

Hemofilter

Autotransfusion

Can. art. 1

Can art. 2

Can venous 1

Can venous 2

---

*i*

Set Up Data

X

1901 — 1902

Data Base Fieldname

1903
1905

| tubing |
| tubing |
| artcansite |
| artcan |
| artcansite2 |
| artcan2 |
| vencansite1 |
| vencan1 |
| vencansite2 |

Save

1904

---

Fig. 19

EP 2 581 846 A1

Fig. 20

2000

2100

Fig. 21

Fig. 22

EP 2 581 846 A1

Fig. 23

Fig. 24

# EP 2 581 846 A1

Europäisches
Patentamt

European
Patent Office

Office européen
des brevets

## EUROPEAN SEARCH REPORT

**Application Number**

EP 11 18 4981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/152544 A1 (WEAVER JAMES WHITT [US] ET AL) 17 June 2010 (2010-06-17) * the whole document * | 1-14 | INV. G06F19/00 A61B5/00 A61M1/36 |
| X | EP 1 721 571 A2 (RANUCCI MARCO [IT]) 15 November 2006 (2006-11-15) * the whole document * | 1-14 | |
| A | US 2010/036676 A1 (SAFDI MICHAEL [US] ET AL) 11 February 2010 (2010-02-11) * paragraph [0294] * | 1 | |
| A | CAPPS S B ET AL: "Body surface area as a predictor of aortic and pulmonary valve diameter", JOURNAL OF THORACIC AND CARDIOVASCULAR SURGERY, MOSBY-YEAR BOOK, INC., ST. LOUIS, MO, US, vol. 119, no. 5, 1 May 2000 (2000-05-01), pages 975-982, XP027314461, ISSN: 0022-5223 [retrieved on 2000-05-01] * abstract * * Section "Comment" * | 2 | |
| A | RL Peverini et al: "Anticoagulation therapy advisor: a decision-support system for heparin therapy during ECMO", Proc Annu Symp Comput Appl Med Care 1992, 8 November 1992 (1992-11-08), pages 567-571, XP002670625, Baltimore Retrieved from the Internet: URL:http://www.ncbi.nlm.nih.gov/pmc/articl es/PMC2248144/ [retrieved on 2012-03-01] * the whole document * | 8 | TECHNICAL FIELDS SEARCHED (IPC) G06F A61B A61M |

The present search report has been drawn up for all claims

| Place of search | Date of completion of the search | Examiner |
|---|---|---|
| The Hague | 2 March 2012 | Philips, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

               

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 18 4981

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2012

| Patent document cited in search report | | Publication date | Patent family member(s) | | Publication date |
|---|---|---|---|---|---|
| US 2010152544 | A1 | 17-06-2010 | US 2010152544 A1 | | 17-06-2010 |
| | | | US 2010152563 A1 | | 17-06-2010 |
| EP 1721571 | A2 | 15-11-2006 | AT 434971 T | | 15-07-2009 |
| | | | EP 1721571 A2 | | 15-11-2006 |
| | | | EP 2092881 A1 | | 26-08-2009 |
| | | | JP 4624301 B2 | | 02-02-2011 |
| | | | JP 2006314800 A | | 24-11-2006 |
| | | | US 2006257283 A1 | | 16-11-2006 |
| | | | US 2009043173 A1 | | 12-02-2009 |
| | | | US 2009043212 A1 | | 12-02-2009 |
| | | | US 2009043219 A1 | | 12-02-2009 |
| US 2010036676 | A1 | 11-02-2010 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LINDERKAMP O. ; VERSMOLD HT. et al.** Estimation and Prediction of Blood Volume in Infants and Children. *Eur J. Pediatr,* 1977, vol. 125, 227-234
- **DUBOIS D ; DUBOIS EF.** *Arch. Intern. Med.,* 1916, vol. 17, 863-871
- **GEORGE SL.** *Cancer Chemother. Rep.,* 1970, vol. 54, 225-235
- **MOSTELLER RD.** *N. in Engl. J. Med.,* 1987, vol. 317, 1098